Europäisches Patentamt

European Patent Office

Office européen des brevets

(11) Veröffentlichungsnummer: **0 229 356 B1**

# EUROPÄISCHE PATENTSCHRIFT

(45) Veröffentlichungstag der Patentschrift: **25.11.92**

(51) Int. Cl.5: **B01D 11/02**, C07C 51/09, C07C 63/26

(21) Anmeldenummer: **86117650.1**

(22) Anmeldetag: **18.12.86**

Die Akte enthält technische Angaben, die nach dem Eingang der Anmeldung eingereicht wurden und die nicht in dieser Patentschrift enthalten sind.

(54) **Mehrstufige Anordnung zur Gegenstromwaschung, deren Anwendung und zugehörige Verfahrensmassnahmen.**

(30) Priorität: **10.01.86 DE 3600522**
**27.02.86 DE 3606259**
**22.11.86 DE 3639958**

(43) Veröffentlichungstag der Anmeldung:
**22.07.87 Patentblatt 87/30**

(45) Bekanntmachung des Hinweises auf die Patenterteilung:
**25.11.92 Patentblatt 92/48**

(84) Benannte Vertragsstaaten:
**BE DE ES FR GB GR IT NL**

(56) Entgegenhaltungen:
**DE-B- 1 263 697**
**DE-C- 855 098**
**DE-C- 878 043**
**US-A- 4 555 385**

(73) Patentinhaber: **Firma AMBERGER KAOLIN-WERKE GMBH**
**Georg Schiffer Strasse 70**
**W-8452 Hirschau(DE)**

Patentinhaber: **HÜLS AKTIENGESELLSCHAFT**
**Paul-Baumann-Strasse 1**
**W-4370 Marl 1(DE)**

(72) Erfinder: **Schoengen, Anton, Dipl.-Ing.**
**In den Espeln 9**
**W-5810 Witten(DE)**
Erfinder: **Schroeder, Heinrich, Dr. Dipl.-Ing.**
**Örtli 5**
**W-4600 Dortmund(DE)**
Erfinder: **Porschen, Jörg**
**Merzenicher Strasse 154**
**W-5160 Düren(DE)**
Erfinder: **Donhauser, Friedrich, Dipl.-Ing.**
**Kolumbusstrasse 62**
**W-8450 Amberg(DE)**

(74) Vertreter: **Richter, Bernhard, Dipl.-Ing. et al**
**Beethovenstrasse 10**
**W-8500 Nürnberg 20(DE)**

EP 0 229 356 B1

## Beschreibung

Die Erfindung betrifft zunächst eine mehrstufige Anordnung zur Gegenstromwaschung von suspendierten Feststoffen, vorzugsweise Kristallen, mit Hilfe von hintereinander geschalteten Hydrozyklonen, sowie zugehörigen Pumpen und Pumpensümpfen und die Vorgenannten Bauelemente verbindenden Leitungen, wobei Zuläufe für die Waschflüssigkeit und die suspendierten Feststoffe, sowie Abläufe für die verunreinigte Waschflüssigkeit und für die gewaschene und eingedickte Suspension vorgesehen sind, wobei ferner die Waschflüssigkeit im Gegenstrom zur Förderrichtung der suspendierten Feststoffe gefördert wird und wobei die suspendierten Feststoffe unter erhöhtem Druck und Temperatur behandelt werden (Oberbegriff des Anspruches 1). Eine solche Anordnung ist in ihrem prinzipiellen Aufbau aus DE-C-3044617 bekannt, die ein Verfahren zur Herstellung von Terephtalsäure aus Dimethylterephtalat als Zwischenprodukt betrifft. Ferner ist eine solche Anordnung vom Prinzip her, einschließlich der zugehörigen mathematischen Grundlagen beschrieben in "Verfahrenstechnik 8 (1974) Nr. 1, Seite 28 - 31" und in "Aufbereitungs-Technik Jahrgang 18 (1977) Seite 395 - 404". Ferner ist eine Gegenstromwaschanlage in einer, das gleiche Verfahren betreffenden weiteren Vorveröffentlichung (DE-C-29 16 197) angegeben. Mit einer solchen Gegenstromwaschung sollen gelöste Verunreinigungen ausgewaschen werden. Diese Verunreinigungen der Aufgabesuspension liegen unterhalb der Trennkorngrenze des Hydrozyklons. Die zu ihrem Auswaschen dienede Waschflüssigkeit kann von unterschiedlicher Art sein. Beispielsweise wird hierfür demineralisiertes Wasser verwendet. Einer der wesentlichen Vorteile der hierzu bekannten Hydrozyklone besteht darin, daß die in der Aufgabesuspension befindlichen Feststoffe klassiert und entsprechend ihrer Klassierung dem Überlauf oder Unterlauf zugeführt werden.

Bei einer Reihe von Anwendungsgebieten besteht die Forderung, daß unter erhöhtem Druck und Temperatur gearbeitet werden muß, wobei aus Prozeßgründen ein bestimmter Temperaturbereich eingehalten werden muß. In dieser Hinsicht ist zum vorstehend genannten und erörterten Stand der Technik nichts zu entnehmen, d.h. er wird diesen Forderungen nicht gerecht.

Der Erfindung liegt die Aufgabe zugrunde, eine Anordnung gemäß dem Eingangs zitierten Oberbegriff des Anspruches 1 so auszugestalten, daß die damit genannten Bedingungen bei Einhaltung eines bestimmten, begrenzten Temperaturbereiches mit einem möglichst geringen apparatemäßigen Aufwand gelöst werden.

Die Lösung dieser Aufgabe wird, ausgehend vom o.g. Oberbegriff des Anspruches 1, darin gesehen, daß die hintereinander geschalteten Hydrozyklone oder hintereinander geschalteten Gruppen aus mehreren parallel geschalteten Hydrozyklonen (beides im folgenden nur noch "Hydrozyklongruppe" genannt) mit Unterläufen und Überläufen und die Pumpensümpfe mit den in sie hineinragenden Pumpen oder Pumpenteilen in einem gemeinsamen, druckfesten und wärmeisolierten Behälter untergebracht sind, und daß dieser Behälter mit dem Zulauf für suspendierte Feststoffe, einem Zulauf und Ablauf für die Waschflüssigkeit und einem Ablauf für die gewaschene und eingedickte Suspension versehen ist. Hiermit sind beispielsweise Drücke in der Größenordnung von 75 bar und Temperaturen in der Größenordnung von 300°C, die in der Praxis auftreten können, beherrschbar. Innerhalb des Behälters herrscht eine gleichmäßige Temperatur, d.h. es entstehen entlang der mehrstufigen Anordnung zur Gegenstromwaschung keine Temperaturabweichungen. Dies ist, wie oben erläutert, wesentlich, da schon geringe Temperaturabweichungen schädlich sein können. Hierzu wird auf die weiteren Ausführungen in dieser Beschreibung, insbesondere die Ausführungen zu den Anwendungsansprüchen 28 bis 32 verwiesen. Die grundsätzlichen Vorteile der Erfindung liegen in einer apparatemäßig einfachen und zugleich im Betrieb zuverlässigen Anordnung zur Verhinderung von Auskristallisationen. Es ist ein gemeinsamer Behälter vorgesehen, der sämtliche Hydrozyklongruppen der gesamten Gegenstromwaschanlage mit den zugehörigen Pumpensümpfen einhaust. Dabei sind gemäß einer bevorzugten Ausführungsform der Erfindung lt. Anspruch 2 alle wesentlichen Teile oder Bereiche der Verbindungsleitungen ebenfalls in dem gemeinsamen, druckfesten und wärmeisolierten Behälter untergebracht. Dies dient zum einen der Sicherheit der mit einer solchen Anordnung arbeitenden Personen. Insbesondere können zum anderen die wesentlichen Zu- und Ableitungen zwischen den einzelnen Gruppen, von den Hydrozyklonen zu den Pumpensümpfen usw. mit eingehaust werden, so daß auch dort die Gefahr von Auskristallisationen vermieden ist. Die noch durch Wärmeabstrahlung entstehenden Wärmeverluste sind sehr gering. Eine alternative Lösung zur Lehre des Anspruches 2 ist im Anspruch 3 enthalten, wonach wesentliche Teile oder Bereiche der Leitungen, oder Teile der Pumpen oder der Pumpenleitungen außerhalb des druckfesten und wärmeisolierten Behälters vorgesehen und, sofern sie die innerhalb eines bestimmten Temperaturbereiches zu haltenden Medien der Gegenstromwaschung führen, wärmeisoliert und/oder fremdbeheizt sind. Bei beiden vorgenannten Ausführungsformen ist in Verbindung mit dem Inhalt des Anspruches 1 insbesondere dafür gesorgt, daß die Temperatur in und an den Bauteilen, die sich im Behälter befinden und die Temperaturen der Gegenstromwaschung im gewünschten Temperaturbereich gehalten werden. Kältebrücken nach außen sind vermieden. Da Auskristallisationen innerhalb der Rohrleitungen somit vermieden sind können auch im Durchmesser kleinere Rohrleitungen verwendet werden. Noch außerhalb des Behälters befindliche Teile oder Rohrleitungen können entsprechend isoliert und/oder fremdbeheizt werden. Mit der Erfindung werden nicht nur die geschilderten funktionellen Vorteile erreicht, sondern es ist eine apparatemäßig sehr kompakte und einfach aufgebaute Anordnung aus dem Behälter und den darin befindlichen Bauteilen geschaffen, die sich insbesondere in der bevorzugten Ausführung nach Anspruch 2 als ein nach außen hin kompakter, z.B. zylindrischer Behälter mit einigen wenigen Rohrleitungen präsentiert. Da die innerhalb des Behälters befindlichen Bauteile nicht isoliert werden müssen und sich innerhalb des unter erhöhtem Druck stehenden Behälters befinden, also auch nicht für sich druckfest gemacht werden müssen, ergeben sich vorteilhafterweise für die Anordnung nach der Erfindung relativ geringe Herstellungskosten. Die Einhaltung der im Oberbegriff angesprochenen Temperaturen und Drücken ist gewährleistet. Andernfalls wäre das angestrebte Ergebnis nicht erreichbar. Es besteht also nicht die Gefahr bzw. der Nachteil eines Druckabfalles von Stufe zu Stufe. Hierzu und hinsichtlich spezieller und besonders vorteilhafter Anwendungsmöglichkeiten der Erfindung wird auf die späteren Ausführungen zu den Ansprüchen 28 und folgende verwiesen. Außerdem besteht durch die Isolierung der Vorteil der Energieeinsparung.

Die Merkmale des Anspruches 4 ermöglichen für Reparaturen oder Auswechslungen eine leichte Zugängigkeit zu den Hydrozyklonen der jeweiligen Gruppe, indem nur der Domdeckel abgenommen und mit den an ihm befestigten Hydrozyklonen nach oben abgehoben werden muß. Wenn hier in der Mehrzahl von Hydrozyklonen gesprochen ist, so geschieht dies nur der sprachlichen Einfachheit halber. Erfaßt ist damit aber auch der Fall, in dem pro Gruppe nur ein Hydrozyklon vorhanden ist.

Die Merkmale des Anspruches 5 dienen zum Ausgleich von Wärmespannungen.

Die Merkmale des Anspruches 6 stellen eine bevorzugte Ausführungsform der Erfindung dar. Sie bewirken zusätzlich zu der vorgesehenen Isolierung eine Konstanthaltung der Temperatur im Behälter. Dies empfiehlt sich besonders dann, wenn der im Behälter einzuhaltende Temperaturbereich sehr gering ist (siehe Ausführungen zu Anspruch 28 und 29).

Die Ansprüche 7 bis 11 befassen sich mit vorteilhaften Ausgestaltungen der Anordnung, die eine möglichst weitgehende Unterbringung der verschiedenen Zu- und Ableitungen im Behälter zum Ziel haben, andererseits aber die Möglichkeit beinhalten, gegen Druck und/oder Temperatur empfindliche Bauteile, wie die Motoren der Pumpen und Rührwerke außerhalb des Behälters vorzusehen (Ansprüche 7 bis 9). Die Ausgestaltung des Behälters und die Unterbringung der Hydrozyklongruppen, Pumpen und Pumpensümpfe ist gemäß den Ansprüchen 10, 11 so gewählt, daß die Stufen der Gegenstromwaschanlage zwischen den beiden Endbereichen des Behälters in diesem hintereinander angeordnet sind und der jeweilige Pumpensumpf der zugehörigen Pumpe und Hydrozyklongruppe unmittelbar zugeordnet, nämlich darunter vorgesehen ist.

Die Ansprüche 12, 13 und 14 beinhalten vorteilhafte Ableitungen der Unterläufe der Hydrozyklongruppen (Anspruch 12) und der Überläufe der Hydrozyklongruppen (Anspruch 13) im natürlichen Gefälle und unter Reduzierunge sowie Vereinfachung der Leitungen, sowie eine Anordnung der Bauteile in den Stufen des Behälters, welche die erforderliche Länge der außerhalb des Behälters vorgesehenen Druckleitungen soweit als möglich reduziert (Anspruch 14). Auch hieraus werden die baulichen und funktionellen Unterschiede der Erfindung zur eingangs erörterten DE-AS 12 63 697 deutlich, in der lediglich von einer Zuführung des Überlaufes des Hydrozyklons in einen nächsten Mischer gesprochen wird. Dies aber ist nicht die Lehre der vorstehend erörterten Ansprüche der Erfindung.

Die Merkmale des Anspruches 15 ergeben, daß die Pegelhöhe in den Pumpensümpfen genau den gewünschten Wert hat, ohne daß heirfür komplizierte Regelungseinrichtungen, radioaktive Meßeinrichtungen (Cäsiumstrahler) und dergleichen notwendig sind. Auch werden von anderen Anwendungsfällen her bekannte Schwimmerregelungen vermieden, die sich leicht mit den hier auszuwaschenden Feststoffen zusetzen würden; insbesondere wenn es sich um auskristallisierende Feststoffe handelt (siehe die Ausführungen zu Anspruch 28 und 29). Die Merkmale des Anspruches 15 wirken besonders vorteilhaft mit der Überlaufanordnung nach Anspruch 13 zusammen.

Es können Fälle eintreten, daß Kristalle, insbesondere die Kristalle der Terephtalsäure, oder Feststoffe einer aschehaltigen Feinstkohle (Anspruch 32) in einer solchen Gegenstromwaschanlage eine unzulässige, zumindest eine nicht erwünschte Größe haben. Es besteht demnach die weitere Aufgabenstellung, dies innerhalb einer solchen Gegenstromwaschanlage zu vermeiden.

Zur Lösung dieser Aufgabe ist zunächst vorgesehen, daß im Verlauf der Gegenstromwaschung ein Aufstromklassierer zwecks Beseitigung von etwaigen Auskristallisationen und/oder gröberen Feststoffteilchen vorgesehen und ebenfalls in dem gemeinsamen, druckfesten und wärmeisolieren Behälter untergebracht ist (Anspruch 16). Aufstromklassierer sind zwar für sich, z.B. zur Trennung der Korngrößen bei Sanden, bekannt. Sie haben aber bei ihrem Einsatz in einer Gegenstromwaschanlage nach der Erfindung den Vorteil, daß damit etwa entstehende gröbere Kristalle oder Feststoffteilchen entfernt werden können. Außerdem beinhaltet dieser Anspruch 16 das weitere Merkmal der Unterbringung des Aufstromklassierers im gemeinsamen, druckfesten und wärmeisolierten Behälter, so daß auch hinsichtlich des Aufstromklassierers die eingangs zu den Hydrozyklonen usw. genannten Bedingungen erfüllt sind.

Anspruch 18 betrifft den Einsatz eines Aufstromklassierers zur Verringerung der Größe von Kristallen der Terephtalsäure, während Anspruch 19 sich mit dem Einsatz eines Aufstromklassierers zur Beseitigung gröberer Kristalle oder Feststoffteilchen aus einer Suspension oder für die Behandlung einer aschehaltigen Feinstkohle befaßt.

Die Erfindung betrifft ferner Verfahrensmaßnahmen zur Gegenstromwaschung bei einer Anordnung nach einem der Ansprüche 1-21. So wird als Verfahren gemäß Anspruch 22 vorgesehen, daß der Zulauf von Waschflüssigkeit und gegebenenfalls der Zulauf eines Filtrates und die Leistung der Pumpen derart aufeinander abgestimmt werden, daß stets mehr Flüssigkeitszulauf zu den einzelnen Pumpensümpfen als Pumpenabzug besteht. Hierdurch wird ein ständiger Überlauf von einem Pumpensumpf zum benachbarten Pumpensumpf erzielt.

Ferner werden Verfahrensmaßnahmen vorgesehen, um die Abläufe (Vorgänge)bei einer solchen Anordnung zur Gegenstromwaschung, insbesondere die sich durch den Einsatz eines Aufstromklassierers sich ergebenden Abläufe (Vorgänge) zu steuern bzw. zu regeln. Hierdurch kann auch eine Klassierung der Produktkörnung mittels des Aufstromklassierers erreicht werden. So sieht Anspruch 23 vor, daß die Menge der pro Zeiteinheit zugeführten und den Aufstrom bildenden sauberen Waschflüssigkeit so eingestellt wird, daß etwaige Auskristallisationen der Terephtalsäure zumindest so weit aufgelöst werden, daß ihre Größe gleich oder kleiner einer bestimmten maximalen Größe ist.

Ferner kann nach Anspruch 24 die Zuführmenge an Aufstromflüssigkeit in Abhängigkeit von der jeweiligen Suspension, sowie deren Verunreinigungsgrad, bzw. der dort entstehenden Auskristallisation geregelt oder gesteuert werden.

Gemäß Anspruch 25 ist es möglich, daß durch Messung der Dichte der Suspension, die sich im Aufstromklassiererbett befindet, und eine entsprechende Veränderung der Waschwassermenge der Auflösegrad und/oder die abzutrennende Partikelgröße beeinflußt werden.

Anspruch 26 sieht eine Regelung oder Steuerung der Suspensionszufuhr und Anspruch 27 ein Verfahren vor, wonach bei konstanter Körnung der Bestandteile der Suspensionszufuhr eine Regelung der Suspensionszufuhr in Abhängigkeit ihrer Dichte erfolgt.

Ferner besteht die spezielle Aufgabenstellung der Schaffung bzw. Zurverfügungstellung einer Gegenstromwaschanlage für suspendierte Feststoffe in gesättigten Lösungen, um bei Drücken, die oberhalb des Dampfdruckes der Waschflüssigkeit bei der Arbeitstemperatur liegen, ein weiteres Kristallisieren der in der Suspension gelösten Feststoffe zum einen mit Sicherheit und zum anderen ebenfalls mit einem vertretbaren apparatemäßigen Aufwand zu verhindern.

Der Lösung dieser Aufgabe dient die Lehre des Anspruches 28, nämlich der Anwendung der hier beanspruchten Anordnung und der hier beanspruchten Verfahrensschritte für die Behandlung von gesättigten Lösungen mit auskristallisierenden suspendierten Feststoffen, zwecks Vermeidung von Auskristallisation. Dem liegt die Erkenntnis zugrunde, daß dies sich als besonders vorteilhaft für die Behandlung von gesättigten Lösungen erweist, die somit suspendierte Feststoffteilchen (Kristalle) enthalten. Bei der Behandlung solcher Lösungen ist es von entscheidender Bedeutung, ein unerwünschtes Auskristallisieren zusätzlicher Feststoffteilchen zu vermeiden. Dieses zusätzliche Auskristallisieren könnte zum einen zur Folge haben, daß sich Rohrleitungen, Behälterwände, Schuten, Stutzen usw. mehr oder weniger schnell verstopfen, und zum anderen ein unzulässiges Auskristallisieren auch von Verunreinigungen verursachen, die z.B. beim Reinigen von Terephtalsäure (siehe Ansprüche 29 bis 31) im Lösungsmittel enthalten sind und an sich durch den Waschprozeß entfernt werden sollen. Um diese Nachteile zu vermeiden, muß sichergestellt sein, daß bei der Gegenstromwäsche eine Unterschreitung des zur Arbeitstemperatur des Lösungsmittels gehörenden Dampfdruckes in jedem Fall vermieden wird. Eine solche Unterschreitung könnte allein z.B. durch den Ansaugvorgang von Pumpen bedingt sein. Sie hätte eine entsprechende Verdampfung des Lösungsmittels mit der Konsequenz des vorgenannten Auskristallisierens zur Folge. Dies aber wird gemäß der Erfindung dadurch verhindert, daß sich der Pumpensumpf, aus dem angesaugt wird, innerhalb des unter Druck stehenden Behälters befindet.

Die gleiche Gefahr besteht auch, wenn es zu einer unzulässigen Erniedrigung der Arbeitstemperatur der Gegenstromwäsche kommt. Die Arbeitstemperatur der Gegenstromwäsche ist z.B. bei der nachstehend näher erläuterten Herstellung von Terephtalsäure entsprechend der DE-PS 30 44 617 bedingt durch die Löslichkeit und Cokristallisationsneigung der in dem Lösungsmittel (Mutterlauge) enthaltenen Verunreinigungen. Solche Verunreinigungen haben die Tendenz, auf den reinen Terephtalkristallen aufzukristallisieren. Die Arbeitstemperatur wird so festgelegt, daß einerseits die Terephtalsäure möglichst nahezu auskristallisiert ist, andererseits aber die Verunreinigungen noch möglichst vollständig gelöst sind. Eine Temperatursenkung hätte in diesem Falle zwangsläufig wiederum zur Folge, daß auch hier die Verunreinigungen in unerwünschter Weise mit den vorgenannten Konsequenzen auskristallisieren würden und zudem noch die vorgenannte Gefahr der Verstopfung auftritt.

Die erfindungsgemäße Gegenstromwäsche ermöglicht es mit vergleichsweise geringem apparativen Aufwand, bei diesen relativ extremen Anforderungen hinsichtlich Druck und Temperatur noch einwandfreie Ergebnisse, d.h. ein "gewaschenes" Produkt mit einem minimalen Gehalt an Verunreinigungen zu erhalten. Dies wird dadurch erreicht, daß es diese Anordnung ohne weiteres ermöglicht, bei einem Druck zu arbeiten, der oberhalb des vorgenannten Dampfdruckes des Lösungsmittels bei der Arbeitstemperatur liegt, so daß die Gefahr einer unzulässigen Verdampfung eindeutig ausgeschlossen werden kann.

Eine bevorzugte Anwendung der Lehre des Anspruches 28 ergibt sich, wie vorstehend bereits erwähnt, aus Anspruch 29. Hinsichtlich Einzelheiten eines möglichen und bevorzugten Verfahrens zur Herstellung von reiner Terephtalsäure aus Dimethylterephtalat als Zwischenprodukt wird auf die beiden eingangs zitierten DE-PS 29 16 197 und 30 44 617 verwiesen. Dabei handelt es sich bei den Feststoffen um Terephtalsäure, bei dem Lösungsmittel (Mutterlauge) um Wasser, bei den Verunreinigungen um Monomethylterephtalat und die Isomeren der Terephtalsäure sowie bei der Wschflüssigkeit um demineralisiertes Wasser. Anstelle von Dimethylterephtalat als Zwischenprodukt (Anspruch 30) kann man auch nach einem weiteren Anspruch der Erfindung von roher Terephtalsäure als Zwischenprodukt ausgehen (Anspruch 31). Dann handelt es sich bei dem Feststoff gleichfalls um Terephtalsäure, bei den Lösungsmitteln wieder um Wasser, Essigsäure oder deren Gemische, bei den Verunreinigungen um Paratoluuelsäure, 4-carboxybenzaldehyd (4-cba) und die Isomeren der Terephtalsäure, sowie alle anderen Verunreinigungen, die in Oxydaten und/oder Hydrierung entstehen. In diesen beiden bevorzugten Anwendungsfällen muß man einen Temperaturbereich von weniger als ± 1° C konstant halten. Auskristallisierungen, z.B. durch Kältebrücken, sind unbedingt zu vermeiden.

Weiterhin kann die Lehre nach einem oder mehreren der Ansprüche 1 bis 27 auch anders angewendet werden, als in den Ansprüchen 28 bis 31 angegeben und in dem Zusammenhang vorstehend näher erläutert wurde. Die Möglichkeit einer solchen, weiteren Anwendung ist Inhalt des Anspruches 32. Danach ist eine solche Anwendung vorgesehen für die Behandlung einer, aschehaltigen Feinstkohle in der Körnung bevorzugt kleiner als 1 mm enthaltenden Kohlewasserstoffsuspension, wobei als Waschflüssigkeit Wasser vorgesehen ist und der Überlauf in Form der in kohlewasserstoffhaltigen Flüssigkeit suspendierten weiter hydrierbaren Feinstkohlefraktion das angestrebte Produkt darstellt. Eine solche Kohlehydrierung findet bei hohen Drücken und Temperaturen statt, so daß auch hierzu die Lösung der eingangs genannten Aufgabe wesentlich für den Erfolg einer solchen Gegenstromwaschung ist.

Hinsichtlich weiterer Merkmale und Vorteile der Erfindung wird sowohl auf den Inhalt der vorstehend ziffernmäßig zitierter Unteransprüche, sowie auf alle weiteren Unteransprüche und ferner auf die nachstehende Beschreibung und die zugehörige Zeichnung von erfindungsgemäßen Ausführungsmöglichkeiten verwiesen. In der Zeichnung zeigt:

Fig. 1: das schematische Fließbild der Gegenstromwäsche,

Fig. 2: die Seitenansicht einer Anordnung nach der Erfindung, ebenfalls schematisch, mit eingezeichneten Bauteilen,

Fig. 3: eine Draufsicht auf Fig. 2,

Fig. 4: einen Schnitt gemäß der Linie A - A in Fig. 2,

Fig. 5: im vergrößerten Maßstab einen Ausschnitt aus der Behälterwandung,

Fig. 6: ein Fließschaltbild analog der Darstellung in den Fig. 1 und 2, jedoch unter Weglassung der dort bereits dargestellten Fließabläufe bzw. Verbindungen, jedoch mit einem zu Beginn der Zuführung der Waschflüssigkeit eingesetzten Aufstromklassierer ,

Fig. 7: einen Schnitt gemäß der Linde VII-VII in Fig. 6,

Fig. 8: ein Fließschaltbild analog Fig. 6, jedoch bei Anordnung des Aufstromklassierers an einer anderen Stelle des Behälters und

Fig. 9: eine schematische Darstellung des Fließablaufes einer Anordnung nach Fig. 6 mit einer Variante des Austrages der verunreinigten Waschflüssigkeit. Dieses Fließschaltbild gilt sinngemäß auch für die Ausführung nach Fig. 8 mit der Maßgabe, daß der Aufstromklassierer an der Stelle gemäß Fig. 8 vorzusehen ist.

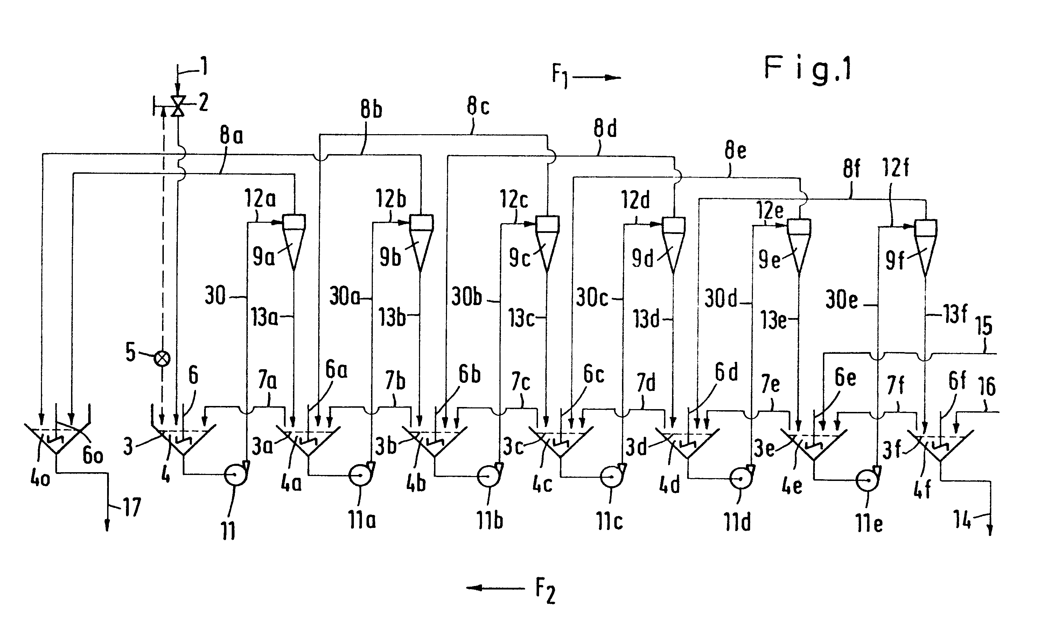

Zunächst wird anhand der Fig. 1 die Förderrichtungen der Fließ- bzw. Stromverläufe usw. in der Gegenstromwaschanlage erläutert. Die verunreinigte Suspension läuft durch die Leitung 1 zu und kann durch ein Ventil 2 in Abhängigkeit vom Flüssigkeitsspiegel 3 eines Pumpensumpfes 4 (Vorstufe) geregelt werden und zwar mittels einer nur schematisch angedeuteten Einrichtung 5, die z.B. mit radioaktiven Strahlen die Pegelhöhe 3 mißt. Außer der verunreinigten Suspension 1 wird in den Pumpensumpf 4 noch der Überlauf 7a des nächstfolgenden Pumpensumpfes 4a geleitet. Der Inhalt des Pumpensumpfes 4 wird über eine Pumpe 11 und eine Leitung 30 dem Zulauf 12a der in Förderrichtung F 1 der Suspension nachgeordneten Hydrozyklongruppe 9a zugeleitet. Deren Unterlauf 13a gelangt in den zugehörigen Pumpensumpf 4a mit Rührwerk 6a. In diesen Pumpensumpf 4a führen in der Förderrichtung F 2 der Waschflüssigkeit der Überlauf 7b des in Förderrichtung F 1 nächstfolgenden Pumpensumpfes 4b und der Überlauf 8c der in Förderrichtung F 1 übernächsten Hydrozyklongruppe 9c. Dies setzt sich entsprechend durch die einzelnen aus Hydrozyklongruppen, Pumpensümpfen mit Pumpen und Rührwerk bestehenden Stufen der Gegenstromwaschanlage fort, wobei stufenweise die Suspension immer wieder eingedickt und damit immer mehr gereinigt wird, bis sie schließlich bei 14 aus dem Pumpensumpf 4f der letzten Stufe ausfließt. Die Waschflüssigkeit wird bei 15 in der Strömungsrichtung F 2 zugegeben und fließt über die Überläufe 7a bis 7e. Bei 16 kann ein Filtrat mit eingegeben werden. Außerdem fließen im freien Fall die Überläufe der Pumpensümpfe 4f, 4e usw. gemäß den Ziffern 7f, 7e usw. in der Förderrichtung F 2. Ebenso wie die vorstehend erläuterten Bauteile werden auch die Unterläufe der Hydrozyklongruppen 9a, 9b, usw. mit 13a, 13b, usw. sowie deren Überläufe mit 8a, 8b, usw. beziffert. Die Unterläufe fließen in die jeweils darunter befindlichen Pumpensümpfe 4a, 4b, usw. In den Pumpensümpfen findet die Vermischung von eingedicktem Zyklonunterlauf und dem darin eingeführten Überlauf statt. Die Überläufe der Hydrozyklone fließen dagegen in Richtung F2 in den jeweils übernächsten Pumpensumpf.

Der in Fig. 1 links gelegene Endbereich beinhaltet nicht nur die eingangs erwähnte Suspensionszufuhr 1, sondern auch die Abfuhr, d.h. den Unterlauf 17 der verunreinigten Waschflüssigkeit aus einem Pumpensumpf 4o, in den die Überläufe 8a und 8b der beiden in Förderrichtung F1 nächstgelegenen Hydrozyklonstufen münden. Der andere, in Fig. 1 rechts gelegene Endbereich beinhaltet die schon erläuterte Abfuhr 14 der gereinigten und eingedickten Suspension und die o.g. Zuleitungen 15, 16.

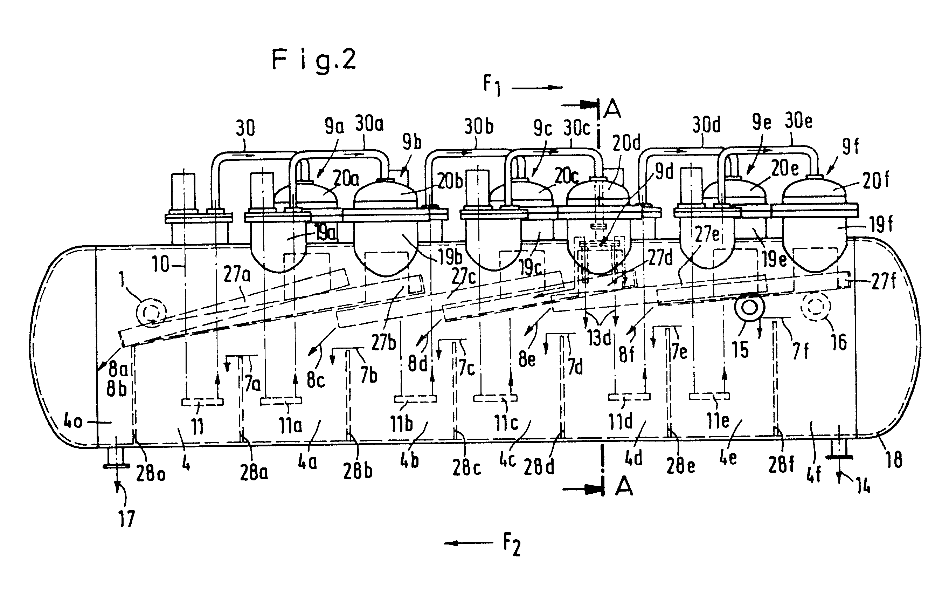

Die in der Fig. 1 erläuterten Elemente dieser Gegenstromwaschanlage sind in den Fig. 2 bis 4 mit den gleichen Ziffern bezeichnet. Ein diese Elemente im wesentlichen umgebender Behälter 18 ist druckfest und wärmeisoliert. Beim Einsatz der Anordnung nach der Erfindung können z.B. Drücke in der Größenordnung von 75 bar und Temperaturen in der Größenordnung von 300° C auftreten. Diese Angaben erfolgen aber nur beispielsweise, ohne daß die Erfindung hierauf beschränkt ist. Jede Hydrozyklongruppe 9 ist in diesem bevorzugten Ausführungsbeispiel in einem Dom angeordnet, der aus einem mit dem Behälter 18 fest verbundenen Unterteil 19a bis 19f und einem damit lösbar verbundenen, bevorzugt angeflanschten Oberteil (Deckel) 20a bis 20f besteht. Nach Lösen der Flanschverbindung und gegebenenfalls der Zuleitung 12 kann der jeweilige Domoberteil 20a bis 20f mit den daran hängenden Hydrozyklonen nach oben zwecks Auswechseln oder Reparatur der Hydrozyklone abgehoben und dann anschließend wieder auf den jeweiligen Domunterteil 19a bis 19f aufgesetzt und damit fest verbunden werden.

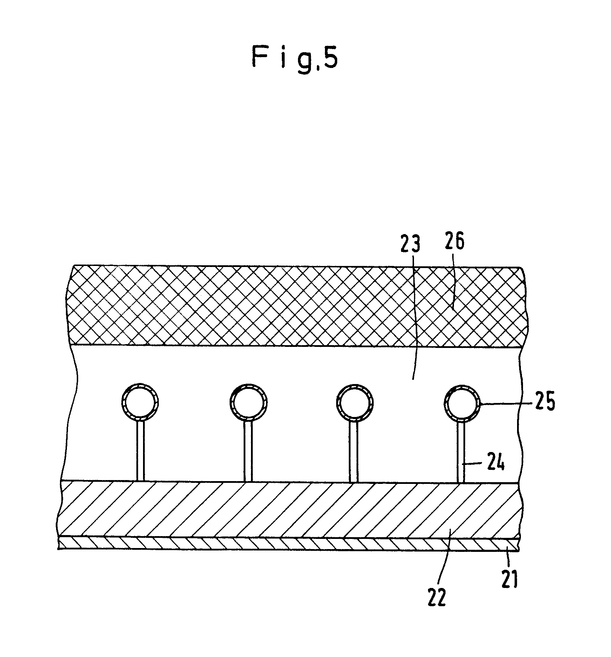

Fig. 5 zeigt in vergrößertem Maßstab einen Ausschnitt aus der Behälterwandung und der Domwandung, bestehend aus einer inneren Schutzschicht 21, z. B. aus Edelstahl 1.4571 oder Titan oder einem anderen korrosionsbeständigen Werkstoff. DIN 1.4571 ist eine international bekannte Bezeichnung. Dies ist ein austenitischer Stahl mit der Anlalyse: Si 1,0; Mn 2,0; P 0,045; S 0,030; Cr 16,50-18,50; Mo 2,00-2,50; Ni 11,00-14,00 und C $\leq$ 0,08. Im Fall eines Kohlenstoffgehaltes kleiner als 0,03 ist ein solcher Stahl stabilisiert, andernfalls muß eine Stabilisierung z. B. durch Titan, erfolgen. Grundsätzlich sind auch Chrom-Nickel-Stähle vom Typ X 10 Cr Ni 18 8 mit austenitischem Gefüge verwendbar. Ähnliche und korrosionstechnisch gleichwertige Stähle sind unter der USA-Bezeichnung 316 L bekannt. Die vorstehenden Angaben sind jedoch nur beispielsweise gemeint, ohne daß die Erfindung hierauf beschränkt ist. An diese innere Schutzschicht 21 schließt sich der tragende Stahlmantel 22 an. Daran folgt in Richtung nach außen ein Luftzwischenraum 23, in dem sich eine von Stegen 24 gehaltene Beheizung 25, z. B. von Dampf durchströmte Rohre, befindet. 26 ist die außen gelegene Isolierschicht. Beheizung und Isolierung sind aufeinander abgestimmt.

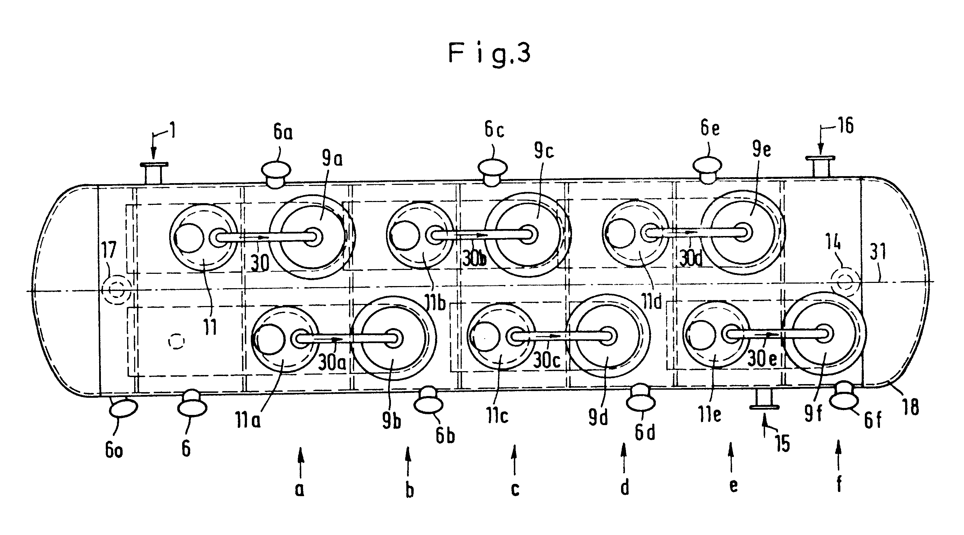

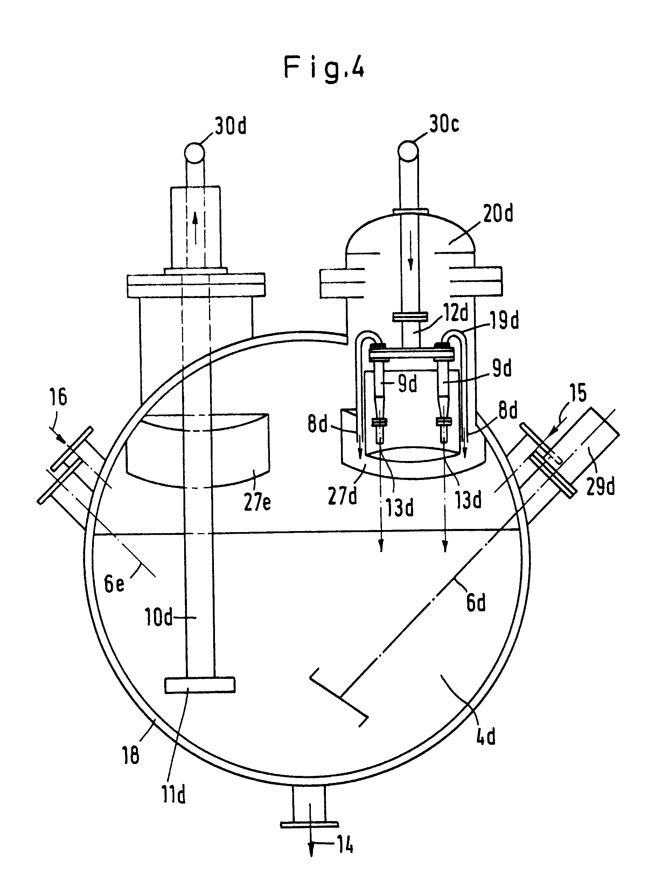

Aus Fig. 2 und auch aus der demgegenüber im größeren Maßstab gezeichneten Fig. 4 zeigt sich, daß die Unterläufe 13a bis 13f und die Überläufe 8a bis 8f der Hydrozyklongruppen und ihre Ableitungen zu den Pumpensümpfen 4 bis 4f sich vollständig innerhalb des Behälters 18 bzw. der Dome 19a bis 19f, 20a bis 20f befinden. Dabei fließen die Unterläufe 13a bis f direkt in die darunter befindlichen Pumpensümpfe 4a bis f. Die Überläufe der Hydrozyklongruppen fließen in darunter befindliche Rinnen, Schuten oder dergleichen 27a bis 27f, welche funktionell den Leitungen 8a bis 8f der Fig. 1 entsprechen. Diese Rinnen, Schuten oder dergleichen sind in der Förderrichtung F 2 abfallend geneigt und so angeordnet, daß sie den Überlauf der Hydrozyklongruppen in dieser Förderrichtung der Waschflüssigkeit im natürlichen Gefälle entweder durch die Rinnen, Schuten oder dergleichen 27b bis 27F in den übernächsten Pumpensumpf bzw. im Fall der Rinne, Schute oder dergleichen 27a in den Pumpensumpf 4o des in Fig. 2 links gelegenen Bereiches fließen lassen. Die einzelnen Pumpensümpfe 4 bis 4f sind durch Trennwände 28 f bis 28a voneinander getrennt, wobei in der Förderrichtung F 2 diese Trennwände jeweils etwas niedriger werden. Wird ferner dafür gesorgt, daß der Zulauf an Waschflüssigkeit 15 und die jeweilige Pumpenleistung so aufeinander abgestimmt sind, daß stets der gesamte Zulauf an Flüssigkeit in den jeweiligen Pumpensumpf größer ist als der Pumpenabzug, so sind die Pumpensümpfe stets bis zum Rand der in der Förderrichtung F 2 der Waschflüssigkeit liegenden Trennwand 28 gefüllt, d.h. die Niveauhöhe der Sümpfe ist immer auf einen gleichbleibenden Wert einreguliert. Die Füllung der Pumpensümpfe hängt nicht nur vom Zulauf 15 an Waschflüssigkeit und gegebenenfalls dem Zulauf an Filtrat 16 ab, sondern der Pumpensumpf wird aus der Kombination des Überlaufes 7 des in Fig. 3 jeweils rechts gelegenen Pumpensumpfes, des Unterlaufes 13 des darüber befindlichen Hydrozyklones und des Überlaufes 8 des in Fig. 1 bzw. 2 nach rechts betrachtet übernächsten Hydrozyklones gefüllt. Diese Gesamtzuflußmenge kann durch Veränderungen des Zulaufes 15 der Waschflüssigkeit und gegebenenfalls des Filtrates 16 gesteuert werden. Diese Füllung wird durch den Abzug der jeweiligen Pumpe des jeweiligen Pumpensumpfes gemindert. Ist der gesamte Zulauf in den jeweiligen Pumpensumpf größer als der Pumpenabzug, so sind die Bedingungen des Anspruches 16 erfüllt. Hierdurch besteht der Vorteil, daß aufwendige Regelungen für den Flüssigkeitsstand der Pumpensümpfe nicht erforderlich sind. Es stellt sich ein interner Überlauf 7a bis 7f in Förderrichtung F 2 über die in der Höhe abnehmenden Trennwände 28f bis 28a zwischen den Pumpensümpfen ein. Dieser Überlauf mündet dann in die Zulaufkammer 4 und wird über die dort vorgesehene Standregelung 5 und 2 mit dem Zulauf 1 der zu waschenden Feststoffsuspension ausgeglichen. Schwimmerregelungen oder dergleichen Mittel sind, wie erwähnt, nicht nötig. Die Trennwand 28o zwischen den Pumpensümpfen 4 und 4o ist allerdings bewußt höher gemacht, da an dieser Stelle kein Überlauf stattfinden soll. Aus den vorgenannten Gründen wird ein Leerlauf der Pumpen vermieden. Die Antriebsmotoren der Pumpen 11 bis 11f und 29 bis 29f der Rührwerke befinden sich bevorzugt außerhalb des Behälters, damit sie nicht durch den im Behälterinnern vorhandenen hohen Druck und die dort gegebene hohe Temperatur angegriffen werden. Die Pumpen können entweder als Tauchpumpen ausgebildet sein und damit vollständig innerhalb des Behälters 18 vorgesehen werden. Auch wäre es möglich, einen Teil der Pumpen an der Behälteraußenseite vorzusehen und dort zu beheizen, wobei die übrigen Teile der Pumpe in den Behälter, nämlich in den zugehörigen Pumpensumpf hineinragen.

Die Fig. 2, 3 zeigen ferner, daß die Pumpen-Druckleitungen 30 bis 30e mit den Zuläufen der in Förderrichtung F 1 der suspendierten Feststoffe nächstfolgenden Hydrozyklongruppe verbunden sind. Auch an dieser Stelle sei darauf hingewiesen, daß zu einer Hydrozyklongruppe entweder nur ein einzelner Hydrozyklon oder mehrere, einander parallel geschaltete Hydrozyklone gehören. Die Pumpen-Druckleitungen sind wärmeisoliert. Wenn auch gemäß Anspruch 3 die Leitungen außerhalb des Behälters vorgesehen und zwecks Vermeidung von Auskristallisationen beheizt, z. B. fremd beheizt, sein können (in der Zeichnung nicht dargestellt), so ist es eine bevorzugte Ausführungsform der Erfindung, die außerhalb des Behälters liegenden Leitungsabschnitte möglichst kurz zu halten, so daß sie sich mit dem größeren Teil ihrer Länge innerhalb des Behälters befinden. Ferner kann gemäß Fig. 3 die Anordnung wie folgt getroffen sein: Beiderseits der senkrechten, sich in Längsrichtung des Behälters 1 erstreckenden Ebene 31 sind hintereinander alternierend eine Pumpe und eine Hydrozyklongruppe angeordnet. In jeder der Stufen liegen, also etwa quer zur Richtung der Längsmittelebene 31, eine Pumpe und eine Hydrozyklongruppe nebeneinander, wobei sich in Fig. 3 links der Hydrozyklongruppe der Stufe a eine Pumpe und rechts der Pumpe der Stufe e noch bei f eine Hydrozyklongruppe befindet. Mit dieser räumlichen Anordnung wird die erforderliche Länge der Pumpen-Druckleitungen 30 bis 30e so gering als möglich gehalten. In den unteren Bereichen dieser Stufen befinden sich die bereits erläuterten Pumpensümpfe. Die Pumpenwellen sind mit 10 beziffert.

Anwendungsmöglichkeiten der Erfindung sind eingangs bereits im Zusammenhang mit den Ansprüchen 17 und 18 erörtert. Eine weitere Anwendungsmöglichkeit der Erfindung, bei der keine gesättigte Lösung mit auskristallisierenden suspendierten Feststoffen vorgesehen ist, wird nachstehend geschildert: Eine Kohlenwasserstoffsuspension, die aschehaltige Feinstkohle in der Körnung kleiner als 1 mm enthält, wird bei hohem Druck (z.B. 60 bar) und hoher Temperatur (z.B. 200° C) mit möglichst wenig Wasser im Gegenstrom nach der bereits beschriebenen Methode getrennt. Die Trennung erfolgt dabei in die aschereichere und auch spezifisch schwerere und gröbere wasserhaltige Fraktion (Unterlauf) einerseits und in die weiter hydrierbare Feinstkohlefraktion (Überlauf) andererseits, die in der kohlenwasserstoffhaltigen Flüssigkeit suspendiert ist. In diesem Anwendungsfall ist der Überlauf das gewünschte, d.h. angestrebte Produkt, während der Unterlauf, nämlich das aschereiche Kohleprodukt mit etwas Wasser, das Abfallprodukt darstellt.

Dieses Beispiel zeigt also zum einen die Gegenstromwaschung von nichtkristallinen Feststoffen und zum anderen, daß die Anordnung nach der Erfindung auch so genutzt werden kann, daß die Überläufe zum gesuchten Endprodukt führen und die Unterläufe zum Abfallprodukt.

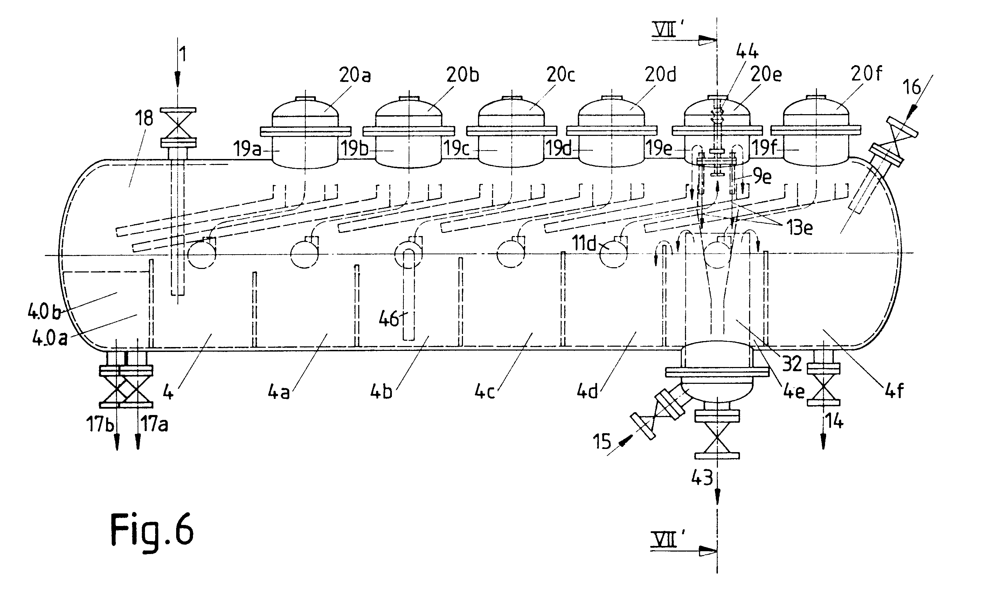

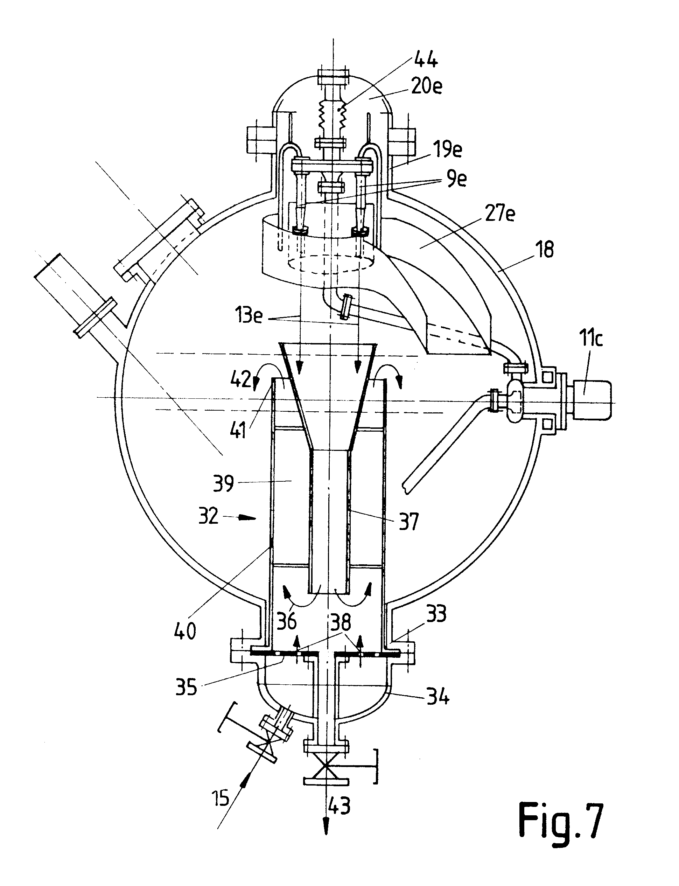

Die schematische Darstellung der Fig. 6 zeigt, unter Weglassung von schon erläuterten Details, einen Aufstromklassierer 32, der im Verlauf dieser Gegenstromwaschung so vorgesehen ist, nämlich im Pumpensumpf 4e und unterhalb der Hydrozyklongruppe 9e, daß der Eingang des frischen Zulaufes an Waschflüssigkeit 15 zugleich das Aufstromwasser dieses Klassierers ist. Dieser Klassierer ist, wie insbesondere Fig. 7 zeigt, im wesentlichen innerhalb des Behälters 18 untergebracht. Soweit er noch aus dem Behälter 18 herausragt, ist ein druckfester und wärmeisolierter Dom 33, 34 vorgesehen, wobei der abnehmbare Domteil 34 es erlaubt, den Aufstromklassierer durch den Stutzen 33 hindurch in den Behälter 18 einzubringen, bzw. aus diesem wieder herauszunehmen. Der Aufstrom 15 fließt durch eine Düsenplatte 35 und trifft auf die eingedickte Suspension der Unterläufe 13e der Hydrozyklongruppe 9. Diese eingedickte Suspension tritt gemäß den Pfeilen 36 aus dem unteren Ende eines den Unterlauf 13e führenden stutzenartigen Rohres 37 aus und wird vom Aufstrom 38 nach oben gerissen und durch das Aufstromklassiererbett 39 in Form eines Ringraumes, der sich zwischen der Klassiereraußenwand 40 und dem Rohr 37 befindet, zum Überlauf 41 des Aufstromklassierers geführt. Soweit es sich um die Anwendung bei einem Verfahren zur Herstellung von Terephthalsäure handelt, können hiermit die zu großen bzw. zu groben Kristalle der Terephthalsäure durch den Aufstrom so weit aufgelöst und damit in ihrer Größe reduziert werden, bis die gewünschte bzw. zulässige Maximalgröße erreicht ist. Dies kann z. B. eine Kristallgröße von $250\mu$ sein. Die Kristalle der gewünschten reduzierten Größe gehen dann über die Überlaufkanten 41 gemäß den Pfeilen 42 in den Pumpensumpf 4e, von dort weiter in den nächstfolgenden Pumpensumpf usw., wie es anhand der Fig. 1, 2 im einzelnen erläutert wurde. Im vorgenannten Anwendungsfall der Herstellung von Terephthalsäure muß der Aufstromklassierer innerhalb der Gegenstomwaschanlage so angeordnet sein (siehe z. B. Fig. 6), daß der Aufstrom von frischer, noch nicht verunreinigter Waschflüssigkeit gebildet wird. Andernfalls würde die angestrebte Abmagerung der gröberen Kristalle nicht oder nur sehr unvollkommen erreicht werden. Auch wenn der wesentliche Teil der groben Kristalle der Terephthalsäure wie vorstehend erläutert abgemagert und mit dem Aufstrom nach oben abgeführt wird, so muß doch ein Austrag bzw. Auslauf 43 am Aufstromklassierer vorgesehen sein.

Es kann nämlich das Aufstromwasser und damit die Lösefähigkeit und gewünschte Kristallverkleinerung nicht immer im exakten Gleichgewicht gehalten werden. Es muß daher die Möglichkeit vorhanden sein, zu grobe Kristalle durch den Auslauf 43 abzuführen. Erwähnt sei, daß die Abmagerung der Kristalle der Terephthalsäure mit frischem, ungesättigten Wasser erfolgen muß, da sonst der Effekt der Auflösung bzw. Abmagerung dieser Kristalle nicht erreicht wird.

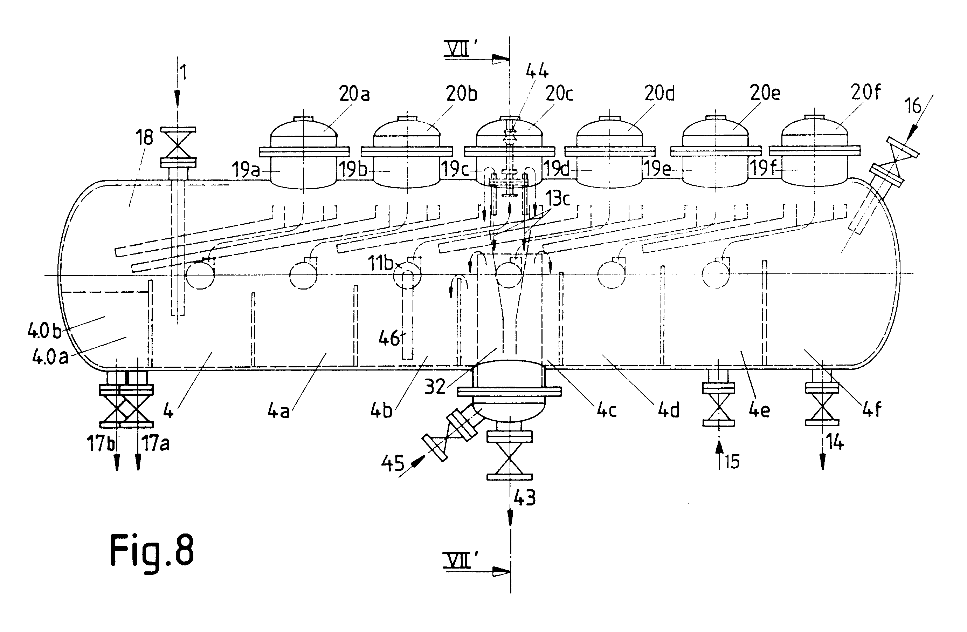

Sofern bei anderen Anwendungsfällen sich in der Suspension Kristalle befinden oder bilden, bzw. Feststoffteilchen vorhanden sind, die jeweils aufgrund ihrer Größe stören aber nicht aufgelöst werden können, können diese Kristalle oder Feststoffteilchen ebenfalls mit Hilfe eines Aufstromklassierers beseitigt werden. Dies zeigt das Ausführungsbeispiel der Fig. 8, wobei der Schnitt VII'-VII' praktisch dem Schnitt VII-VII der Fig. 6 und damit der Fig. 7 entspricht. Auch hier ist die Entleerungsöffnung 43 für den Austrag der auszuscheidenden Kristalle oder Feststoffteilchen vorgesehen. Nur ist es hier nicht notwendig, frisches, ungesättigtes Aufstromwasser zu verwenden. Der Aufstromklassierer kann daher an beliebiger Stelle des Fließablaufes im Behälter 18 vorgesehen sein, z. B. wie Fig. 8 zeigt im Pumpensumpf 4c. In diesem Anwendungsfall erfolgt keine Auflösung oder Abmagerung von Kristallen, sondern nur eine Klassierung von zu groben Teilchen oder Körnern, die auch durch eine verschmutzte Flüssigkeit erfolgen kann. Bevorzugt wird man aber auch für eine Klassierung den Aufstromklassierer im Pumpensumpf 4e, d.h. an der Stelle vorsehen, an der frische Waschflüssigkeit zugeführt wird. Unter weitgehender Benutzung der Bezifferung aus Fig. 7 wird die Funktion des Aufstromklassierers im Ausführungsbeispiel der Fig. 8 wie folgt erläutert:

Bei 45 wird Aufstromwasser zugeführt. Aus der Hydrozyklongruppe 9c fließt der Unterlauf 13c in den Rohrstutzen 37 und wird nach dem Austritt am unteren Rohrstutzenende vom Aufstrom 38 erfaßt, wobei die Klassierung erfolgt. Nur werden in diesem Beispiel die zu großen bzw. groben Kristalle oder Feststoffteilchen nicht teilweise aufgelöst, sondern fallen nach unten durch und treten durch den Entleerungsstutzen 43 aus. Der Überlauf 42 geht in den Pumpensumpf 4c, in dem sich der Aufstromklassierer befindet. Im übrigen ist der Ablauf der Gegenstromwaschung wie anhand der Fig. 1, 2 beschrieben. Die verschiedenen Möglichkeiten der Anordnung des Aufstromklassierers im Behälter 18 sind vorstehend bereits erläutert. Sofern es darum geht, zu grobe Teilchen aus einer aschehaltigen Feinstkohle durch Klassierung zu entfernen, empfiehlt es sich, ihn so weit als möglich an der Ausgangsseite der Waschflüssigkeit, d.h. bevorzugt im Pumpensumpf 4a vorzusehen.

Die Hydrozyklongruppe kann über eine Faltenbalg 44 elastisch im Deckel (20e bzw. 20c) des jeweiligen Domes aufgehängt sein. Dies hat den Vorteil, daß hierdurch Wärmespannungen aufgenommen, bzw. ausgeglichen werden können. Dieser Faltenbalg oder ein anderes elastisches Dehnstück hält den Hydrozyklonverteiler. Außerdem kann durch einen solchen Faltenbalg hindurch ein Zugang (Rohrleitung) zum Verteiler hin durchgeführt werden.

Zur Steuerung bzw. Regelung wird noch darauf hingewiesen, daß man durch Messung der Suspensionsdichte im Aufstromklassiererbett 39 (siehe Fig. 7) und eine entsprechende Veränderung der Aufstromwassermenge den Auflösegrad und/oder die abzutrennende Partikelgröße der Suspension beeinflussen kann.

Um eine gleichmäßige Körnung im Endprodukt zu erreichen, genügt im Fall einer konstanten Körnung der bei 1 zugeführten Suspension die Regelung der Suspensionszufuhr in Abhängigkeit von der Dichte dieser Suspension, die gemessen werden kann.

Fig. 8 zeigt noch eine Saugrohrleitung 46, die an jeder der Pumpen (hier Pumpe 11b) angebracht ist (im einzelnen nicht dargestellt). Diese Leitung dient dazu, die Suspension auch bei niederem Flüssigkeitsstand aus dem Sumpf abpumpen zu können.

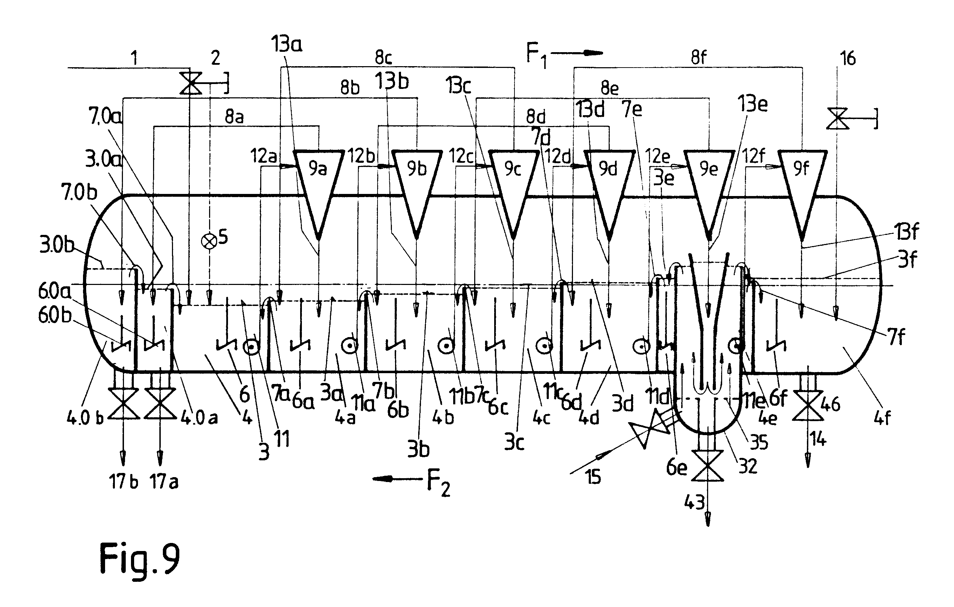

Fig. 9 zeigt zum besseren Verständnis ein Fließschaltbild der Ausführung nach Fig. 6, wobei im wesentlichen die Darstellung und insbesondere auch die Bezifferung der Fig. 1 übernommen wurde. Fig. 9 zeigt außerdem noch die Variante zweier getrennter Unterläufe 17a und 17b der verunreinigten Waschflüssigkeit.

Die Beispiele der Fig. 6 bis 9 zeigen ferner, daß in der Stufe 0 die Kammer bzw. der Pumpensumpf 4o in zwei Kammern bzw. Sümpfe 4.o a und 4.o b unterteilt sein kann. Einzelheiten dieser Aufteilung sind insbesondere Fig. 9 und der zugehörigen Bezifferung zu entnehmen. Hiermit kann man die Überläufe 8a und 8b der Hydrozyklonstufen 9a und 9b getrennt auffangen und damit getrennt verschiedenen Weiterverarbeitungsstufen zuführen. Dies kann deswegen von Vorteil sein, da die Überläufe unterschiedliche Verunreinigungsgrade aufweisen. Der Überlauf 8a ist höher verunreinigt als der Überlauf 8b. Der Überlauf 8a der Hydrozyklongruppe 9a führt also in den Pumpensumpf 4.o a. Diesem ist ein gesonderter Unterlauf 17a zugeordnet. Der Überlauf 8b der Hydrozyklongruppe 9b führt dagegen in den Pumpensumpf 4.o b, dessen Unterlauf der verunreinigten Waschflüssigkeit mit 17b beziffert ist.

Generell ist für alle Ausführungsbeispiele zu sagen, daß bei 15 frische Waschflüssigkeit bzw. Waschwasser zugeführt werden muß. Bei 16 kann eine Verdünnungsflüssigkeit (Filtrat) dann zugeführt werden, wenn dies erforderlich ist, um die Suspension in der Kammer bzw. Sumpf 4f pumpfähig zu machen, d.h. entsprechend zu verdünnen.

Sofern ein Klassierer vorgesehen ist, ist im Ausführungsbeispiel der Fig. 6 die Aufstromflüssigkeit mit der Waschflüssigkeit identisch. Dagegen erfolgt im Beispiel der Fig. 8 bei 15 die Zufuhr der Waschflüssigkeit und bei 45 die Zufuhr einer gesonderten Aufstromflüssigkeit bzw. Aufstromwassers.

## Patentansprüche

1. Mehrstufige Anordnung zur Gegenstromwaschung von suspendierten Feststoffen, vorzugsweise Kristallen, mit Hilfe von hintereinander geschalteten Hydrozyklonen (9a - 9f) sowie zugehörigen Pumpen (11-11e) und Pumpensümpfen (4 - 4f) und die vorgenannten Bauelemente verbindenden Leitungen, wobei Zuläufe für die Waschflüssigkeit (15) und die suspendierten Feststoffe (1), sowie Abläufe für die verunreinigte Waschflüssigkeit (17; 17a, 17b) und für die gewaschene und eingedickte Suspension (14) vorgesehen sind, wobei ferner die Waschflüssigkeit im Gegenstrom (F2) zur Förderrichtung (F1) der suspendierten Feststoffe gefördert wird und wobei die suspendierten Feststoffe unter erhöhtem Druck und Temperatur behandelt werden, dadurch gekennzeichnet, daß die hintereinander geschalteten Hydrozyklone (9a-9f) oder hintereinander geschalteten Gruppen aus mehreren parallel geschalteten Hydrozyklonen (9a - 9f) (beides im folgenden nur noch "Hydrozyklongruppe" genannt) mit Unterläufen (13a - 13f) und Überläufen (8a - 8f) und die Pumpensümpfe (4 - 4f) mit den in sie hineinragenden Pumpen (11 - 11e) oder Pumpenteilen in einem gemeinsamen, druckfesten und wärmeisolierten Behälter (18) untergebracht sind, und daß dieser Behälter mit dem Zulauf (1) für suspentierte Feststoffe, einem Zulauf (15) und Ablauf (17) für die Waschflüssigkeit und einem Ablauf (14) für die gewaschene und eingedickte Suspension versehen ist.

2. Anordnung nach Anspruch 1, dadurch gekennzeichnet, daß die wesentlichen Teile oder Bereiche der Leitungen ebenfalls in dem gemeinsamen, druckfesten und wärmeisolierten Behälter (18) untergebracht sind.

3. Anordnung nach Anspruch 1, dadurch gekennzeichnet, daß wesentliche Teile oder Bereiche der Leitungen, oder Teile der Pumpen oder der Pumpenleitungen außerhalb des druckfesten und wärmeisolierten Behälters (18) vorgesehen und, sofern sie die innerhalb eines bestimmten Temperaturbereiches zu haltenden Medien der Gegenstromwaschung führen, wärmeisoliert und/oder fremdbeheizt sind.

4. Anordnung nach einem der Ansprüche 1 bis 3, dadurch gekennzeichnet, daß der Behälter (18) mit druckfesten und wärmeisolierten Domen (19a - 19f) mit zugehörigen und abnehmbaren Deckeln (20a - 20f) versehen ist, wobei am jeweiligen Deckel die zu einer Gruppe (9a - 9f) gehörenden Hydrozyklone befestigt und im zugehörigen Dom untergebracht sind.

5. Anordnung nach Anspruch 4, dadurch gekennzeichnet, daß die Hydrozyklone bzw. Hydrozyklongruppen (9a - 9f) im Dom mittels dehnbarer Teile, z.B. Faltenbälge (44), befestigt sind.

6. Anordnung nach einem der Ansprüche 1 bis 5, dadurch gekennzeichnet, daß die Wandungen des Behälters und ggfls. der Dome von außen beheizbar (25) sind.

7. Anordnung nach einem der Ansprüche 1 bis 6, dadurch gekennzeichnet, daß die Überläufe (8a - 8f) und die Unterläufe (13a - 13f) der Hydrozyklone bzw. Hydrozyklongruppen (9a - 9f) innerhalb des Behälters (18) oder dessen Dome (19a - 19f) mit den Pumpensümpfen (4 - 4f) in Verbindung stehen.

8. Anordnung nach einem der Ansprüche 1 bis 7, dadurch gekennzeichnet, daß zumindest die Motoren der Pumpen (11 bis 11e) und teilweise die Pumpen-Druckleitungen (30 bis 30e) sich außerhalb des Behälters (18) befinden, wobei die Pumpendruckleitungen mit den Zuläufen (12a bis 12f) der in Förderrichtung (F1) der suspendierten Feststoffe nächstfolgenden Hydrozyklongruppen (9a bis 9f) verbunden sind.

9. Anordnung nach einem der Ansprüche 1 bis 8, dadurch gekennzeichnet, daß jedem Pumpensumpf (4c bis 4f) ein Rührwerk (6c bis 6f) zugeordnet ist, dessen Motor (29c bis 29f) sich außerhalb des Behälters (18) befindet.

10. Anordnung nach einem der Ansprüche 1 bis 9, dadurch gekennzeichnet, daß in einem langgestreckten Behälter (18) in einem Endbereich der Zulauf (1) der suspendierten Feststoffe und der abgehende Überlauf (17) der Waschflüssigkeit, sowie im anderen Endbereich die Zugabe (15) der Waschflüssigkeit und der Abgang bzw. Auslauf (14) der gewaschenen und eingedickten Feststoffsuspension vorgesehen sind, wobei sich zwischen den Endbereichen hintereinander geschaltet die Stufen der Gegenstromwaschanlage befinden.

11. Anordnung nach Anspruch 10, dadurch gekennzeichnet, daß in den Stufen (a bis e) des Behälters (18) nebeneinander, d.h. in einer etwa quer zur Längsmittelebene (31) des Behälters verlaufenden Ebene, je eine Hydrozyklongruppe (9a bis 9e) oder der diese Hydrozyklongruppe enthaltende Dom und je eine Pumpe (11 bis 11e) mit Rührwerk (6a bis 6e) nebeneinander angeordnet sind, wobei sich im unteren Bereich jeder Stufe der Pumpensumpf (4a bis 4e) befindet, der durch Querwände (28a bis 28f) von den jeweils benachbarten Pumpensümpfen getrennt ist.

12. Anordnung nach Anspruch 11, dadurch gekennzeichnet, daß der Unterlauf (13a bis 13f) der Hydrozyklongruppe (9a bis 9f) jeder Stufe direkt nach unten in den darunter befindlichen Pumpensumpf (4a bis 4f) abfließt.

13. Anordnung nach Anspruch 11 oder 12, dadurch gekennzeichnet, daß der Überlauf (8a bis 8f) der Hydrozyklongruppe (9a bis 9f) jeder Stufe direkt nach unten in eine Rinne, Schute oder dergleichen (27a bis 27f) abfließt, die so geneigt und angeordnet ist, daß sie den Überlauf in Förderrichtung (F2) der Waschflüssigkeit im natürlichen Gefälle zum übernächsten Pumpensumpf bzw. zum Pumpensumpf (4o) des Waschflüssigkeitsaustrittes fließen läßt.

14. Anordnung nach einem der Ansprüche 11 bis 13, dadurch gekennzeichnet, daß in den Abschnitten bzw. Stufen des Behälters (18), die sich in dessen Längsrichtung hintereinander befinden, die Pumpen (11 bis 11e) und Hydrozyklongruppen (9a bis 9f) beiderseits einer senkrechten Längsmittelebene (31) des Behälters angeordnet sind, wobei die Längsrichtung des Behälters alternierend eine Pumpe und eine Hydrozyklongruppe, bzw. der sie aufnehmende Dom vorgesehen sind, und daß jeweils die Druckleitung (30 bis 30e) einer Pumpe mit dem Zulauf (12a bis 12f) der in Förderrichtung (F1) der suspendierten Feststoffe benachbarten Hydrozyklongruppe bzw. Dom verbunden ist.

15. Anordnung nach einem der Ansprüche 11 bis 14, dadurch gekennzeichnet, daß die Höhe der Trennwände (28a bis 28f) zwischen den Pumpensümpfen in Förderrichtung (F2) der Waschflüssigkeit abnimmt und damit einen Überlauf von einem Pumpensumpf zu dem in dieser Förderrichtung (F2) benachbarten Pumpensumpf ermöglicht.

16. Anordnung nach einem der Ansprüche 1 bis 15, dadurch gekennzeichnet, daß im Verlauf der Gegenstromwaschung ein Aufstromklassierer (32) zwecks Beseitigung von etwaigen Auskristallisationen und/oder gröberen Feststoffteilchen vorgesehen und ebenfalls in dem gemeinsamen, druckfesten und wärmeisolierten Behälter (18) untergebracht ist.

17. Anordnung nach Anspruch 16, dadurch gekennzeichnet, daß der untere den Zulauf der Waschflüssigkeit und den Austrag (43) aufweisende Bereich des Aufstromklassierers (32) in einem Dom (33, 34) des Behälters angeordnet ist, wobei der Deckel (34) des Dom abnehmbar ist, und daß auch dieser Dom (33, 34) druckfest und wärmeisoliert ist.

18. Anordnung nach Anspruch 16 oder 17, dadurch gekennzeichnet, daß bei einem Verfahren zur Herstellung von Terephtalsäure der Aufstromklassierer (32) am Eingang der Waschflüssigkeit (15) vorgesehen ist, daß der Zufluß der sauberen Waschflüssigkeit (15) an dessen Aufströmöffnung angeschlossen ist und den Aufstrom des Klassierers bildet, daß der Unterlauf (13e) des zugehörigen, darüber befindlichen Hydrozyklons bzw. Hydrozyklongruppe (9e) zu dem Zulauf des Aufstromklassierers geführt ist, daß der Überlauf (42) des Aufstromklassierers in den zu ihm gehörenden Pumpensumpf (4e) fließt, von dort weitergeleitet wird und daß der Austrag (43) des Aufstromklassierers geschlossen ist.

19. Anordnung nach Anspruch 16 oder 17, dadurch gekennzeichnet, daß für die Behandlung von gesättigten Lösungen mit auskristallisierenden suspendierten Feststoffen, oder für die Behandlung einer aschehaltigen Feinstkohle der Aufstromklassierer (32) im Behälter bevorzugt am Ausgang der verunreinigten Waschflüssigkeit vorgesehen ist, daß sein Aufstrom von einer gesondert zugeführten Waschflüssigkeit (45) gebildet ist und daß der Austrag (43) des Aufstromklassierers zwecks Ableitung der klassierten Kristalle oder Feststoffteilchen geöffnet ist.

20. Anordnung nach Anspruch 18 oder 19, dadurch gekennzeichnet, daß der Aufstromklassierer (32) ein Gehäuse (40) mit Überlaufkante (41) und Boden (34) aufweist und unterhalb des Unterlaufes (13a bis 13e) eines Hydrozyklons bzw. einer Hydrozyklongruppe angeordnet ist und daß sich im Boden (34) der Zulauf (15) für die Waschflüssigkeit und der Austragstutzen (43) befinden und daß der Überlauf (42) des Aufstromklassierers (32) oberhalb des ihm zugeordneten Pumpensumpfes über die Überlaufkante (41) fließt.

21. Anordnung nach einem der Ansprüche 1 bis 20, dadurch gekennzeichnet, daß die in Strömungsrichtung der Waschflüssigkeit am Ende des Behälters liegende Stufe (o) in zwei Kammern bzw. Pumpensümpfen (4.o a und 4.o b) unterteilt ist, wobei zu jedem dieser Pumpensümpfe ein gesonderter Unterlauf (17a, 17b) der verunreinigten Waschflüssigkeit gehört und daß die Überläufe (8a und 8b) der Hydrozyklonstufen (9a, 9b) jeweils in den zugehörigen Pumpensumpf (4.o a bzw. 4.o b) geleitet werden.

22. Verfahren zum Betrieb einer Anordnung nach einem der Ansprüche 1 bis 21, dadurch gekennzeichnet, daß der Zulauf von Waschflüssigkeit (15) und gegebenenfalls der Zulauf eines Filtrates (16) und die Leistung der Pumpen (11a - 11e) derart aufeinander abgestimmt werden, daß stets mehr Flüssigkeitszulauf (7b - 7f; 13a - 13f; 8c - 8f; 15 und ggfls. 16) zu den einzelnen Pumpensümpfen (4a - 4f) als Pumpenabzug besteht.

23. Verfahren nach Anspruch 22, dadurch gekennzeichnet, daß die Menge der pro Zeiteinheit zugeführten und den Aufstrom bildenden sauberen Waschflüssigkeit so eingestellt wird, daß etwaige Auskristallisationen der Terephthalsäure zumindest so weit aufgelöst werden, daß ihre Größe gleich oder kleiner einer bestimmten maximalen Größe ist.

24. Verfahren nach Anspruch 23, dadurch gekennzeichnet, daß die Zuführmenge an Aufstromflüssigkeit in Abhängigkeit von der jeweiligen Suspension, sowie deren Verunreinigungsgrad, bzw. der dort entstehenden Auskristallisation geregelt oder gesteuert wird.

**25.** Verfahren nach Anspruch 23 oder 24, dadurch gekennzeichnet, daß durch Messung der Dichte der Suspension, die sich im Aufstromklassiererbett (39) befindet, und eine entsprechende Veränderung der Waschwassermenge der Auflösegrad und/oder die abzutrennende Partikelgröße beeinflußt werden.

**26.** Verfahren nach Anspruch 23 oder 24, gekennzeichnet durch eine Regelung oder Steuerung der Suspensionszufuhr.

**27.** Verfahren nach Anspruch 26, dadurch gekennzeichnet, daß bei konstanter Körnung der Bestandteile der Suspensionszufuhr eine Regelung der Suspensionszufuhr in Abhängigkeit ihrer Dichte erfolgt.

**28.** Anwendung einer Anordnung nach einem der Ansprüche 1 bis 21 und/oder eines Verfahrens nach einem der Ansprüche 22 bis 27 für die Behandlung von gesättigten Lösungen mit auskristallisierenden suspendierten Feststoffen, zwecks Vermeidung von Auskristallisation.

**29.** Anwendung nach Anspruch 28 bei einem Verfahren zur Herstellung von reiner Terephthalsäure.

**30.** Anwendung nach Anspruch 29 bei einem Verfahren zur Herstellung von reiner Terephthalsäure aus Dimethylterephthalat als Zwischenprodukt.

**31.** Anwendung nach Anspruch 29 bei einem Verfahren zur Herstellung von reiner Terephthalsäure aus roher Terephthalsäure als Zwischenprodukt.

**32.** Anwendung einer Anordnung nach einem der Ansprüche 1 - 21 und/oder eines Verfahrens nach einem der Ansprüche 22 bis 27 für die Behandlung einer, aschehaltigen Feinstkohle in der Körnung bevorzugt kleiner als 1 mm enthaltenden Kohlewasserstoffsuspension, wobei als Waschflüssigkeit Wasser vorgesehen ist und der Überlauf in Form der in kohlewasserstoffhaltigen Flüssigkeit suspendierten weiter hydrierbaren Feinstkohlefraktion das angestrebte Produkt darstellt.

**Claims**

**1.** Multi-stage arrangement for counter-current washing of suspended solids, preferably crystals, with the help of hydrocyclones (9a to 9f) connected in series as well as associated pumps (11 to 11e) and pump sumps (4 to 4f) and ducts connecting the aforesaid components, wherein feeds for the washing liquid (15) and the suspended solids (1) as well as outlets for the contaminated washing liquid (17; 17a, 17b) and for the washed and concentrated suspendion (14) are provided, wherein further the washing liquid is conveyed in counter-current (F2) to the conveying direction (F1) of the suspended solids and wherein the suspended solids are treated under raised pressure and temperature, characterised thereby that the hydrocyclones (9a to 9f) connected in series or groups, which are connected in series, of several hydrocyclones (9a to 9f) connected in parallel (both called only "hydrocyclone groups" in the following) together with downward runs (13a to 13f) and upward runs (8a to 8f) and the pump sumps (4 to 4f) together with the pumps (11 to 11e), or pump parts, projecting into them are accommodated in a common pressure-resistant and thermally insulated container (18), and that this container is provided with the feed (1) for suspended solids, a feed (15) and an outlet (17) for the washing liquid and an outlet (14) for the washed and concentrated suspension.

**2.** Arrangement according to claim 1, characterised thereby that the essential parts or regions of the ducts are likewise accommodated in the common, pressure-resistant and thermally insulated container (18).

**3.** Arrangement according to claim 1, characterised thereby that essential parts or regions of the ducts, or parts of the pumps or of the pump ducts are provided outside the pressure-resistant and thermally insulated container (18) and, sofar as they conduct the media, which are to be kept within a certain temperature range, of the counter-current washing, are thermally insulated and/or externally heated.

**4.** Arrangement according to one of the claims 1 to 3, characterised thereby that the container (18) is provided with pressure-resistant and thermally insulated domes (19a to 19f) with associated and removable covers (20a to 20f), wherein the hydrocyclones belonging to a group (9a to 9f) are fastened at the respective cover and accommodated in the associated dome.

**5.** Arrangement according to claim 4, characterised thereby, that the hydrocyclones or hydrocyclone groups (9a to 9f) are fastened in the dome by means of extensible parts, for example bellows (44).

6. Arrangement according to one of claims 1 to 5, characterised thereby, that the walls of the container and in a given case of the domes are externally heatable (25).

7. Arrangement according to one of the claims 1 to 6, characterised thereby, that the upward runs (8a to 8f) and the downward runs (13a to 13f) of the hydrocyclones or hydrocyclone groups (9a to 9f) within the container (18) or the domes (19a to 19f) thereof stand in connection with the pump sumps (4 to 4f).

8. Arrangement according to one of the claims 1 to 7, characterised thereby, that at least the motors of the pumps (11 to 11e) and part of the pump pressure ducts (30 to 30e) are disposed outside the container (18),wherein the pump pressure ducts are connected with the feeds (12a to 12f) of the hydrocyclone group (9a to 9f) next following in the conveying direction (F1) of the suspended solids.

9. Arrangement according to one of claims 1 to 8, characterised thereby, that associated with each pump sump (4a to 4f) is a stirring mechanism (6c to 6f), the motor (29c to 29f) of which is disposed outside the container (18).

10. Arrangement according to one of claims 1 to 9, characterised thereby, that in the case of an elongate container (18) the feed (1) for the suspended solids and the departing overflow (17) for the washing liquid are provided in one end region and the feed (15) for the washing liquid and the discharge or outlet (14) for the washed and concentrated solids suspension are provided in the other end region, wherein the stages, connected one after the other, of the counter-current washing plant are disposed between the end regions.

11. Arrangement according to claim 10, characterised thereby, that in each of the stages (a to e) of the container (18) a respective hydrocyclone group (9a to 9e) or the dome containing this hydrocyclone group and a respective pump (11 to 11e) with stirring mechanism (6a to 6e) are arranged adjacent, i.e. in a plane extending approximately transversely to the longitudinal centre plane (31) of the container, to one another, wherein disposed in the lower region of each stage is the pump sump (4a to 4e), which is separated from the respectively adjacent pump sump by transverse walls (28a to 28f).

12. Arrangement according to claim 11, characterised thereby, that the downward run (13a to 13f) of the hydrocyclone group (9a to 9f) of each stage discharges directly downwards into the pump sump (4a to 4f) disposed thereunder.

13. Arrangement according to claim 11 or 12, characterised thereby, that the upward run (8a to 8f) of the hydrocyclone group (9a to 9f) of each stage discharges directly downwards into a gutter, chute or the like (27a to 27f), which is so inclined and arranged that it permits the overflow to flow in conveying direction (F2) of the washing liquid in natural fall to the next-but-one pump sump or to the pump sump (40) of the washing liquid outlet.

14. Arrangement according to one of claims 11 to 13, characterised thereby, that in the sections or stages, which are disposed one behind the other in the longitudinal direction of the container (18), of the container the pumps (11 to 11e) and hydrocyclone groups (9a to 9f) are arranged at both sides of a vertical longitudinal centre plane (31) of the container, wherein in the longitudinal direction of the container one pump and one hydrocyclone group, or the dome receiving it, are provided in alternation, and that each time the pressure duct (30 to 30e) of a pump is connected with the feed (12a to 12f) of the hydrocyclone group or dome adjacent in the conveying direction (F1) of the suspended solids.

15. Arrangement according to one of claims 11 to 14, characterised thereby, that the height of the separating walls (28a to 28f) between the pump sumps reduces in the conveying direction (F2) of the washing liquid and thus enables an overflow from one pump sump to the pump sump adjacent in this conveying direction (F2).

16. Arrangement according to one of claims 1 to 15, characterised thereby, that in the course of the counter-current washing there is provided an upstream classifier (32) for the purpose of removing possible crystallisations and/or coarser solid particles and is likewise accommodated in the common pressure-resistant and thermally insulated container (18).

17. Arrangement according to claim 16, characterised thereby, that the lower region, having the feed of the washing liquid and the discharge (43), of the upstream classifier (32) is arranged in a dome (33, 34) of the container, wherein the cover (34) of the dome is removable, and that this dome (33, 34) is pressure-resistant and thermally insulated.

**18.** Arrangement according to claim 16 or 17, characterised thereby, that in the case of a method for the production of terephthalic acid the upstream classifier (32) is provided at the inlet of the washing liquid (15), that the inflow of the clean washing liquid (15) is connected to the upstream opening thereof and forms the upstream flow of the classifier, that the downward run (13e) of the associated hydrocyclone, or hydrocyclone group (9e), disposed thereabove is led to the feed of the upstream classifier, that the overflow (42) of the upstream classifier flows into the pump sump (4e) belonging to it and that the discharge (43) of the upstream classifier is closed.

**19.** Arrangement according to claim 16 or 17, characterised thereby, that for the treatment of saturated solutions with suspended solids which crystallise out or for the treatment of an ash-containing fine coal the upstream classifier (32) is provided in the container preferably at the outlet of the contaminated washing liquid, that its upstream flow is formed by a separately fed washing liquid (45) and that the discharge (43) of the upstream classifier is open for the purpose of discharging the screened crystals or solid particles.

**20.** Arrangement according to claim 18 or 19, characterised thereby, that the upstream classifier (32) has a housing (40) with overflow edge (41) and base (34) and is arranged below the downward run (13a to 13e) of a hydrocyclone or of a hydrocyclone group and that disposed in the base (34) are the feed (15) for the washing liquid and the discharge stub pipe (43) and that the overflow (42) of the upstream classifier (32) flows above the pump sump, which is associated therewith, by way of the overflow edge (41).

**21.** Arrangement according to claims 1 to 20, characterised thereby, that the stage (o) lying at the end of the container in the flow direction of the washing liquid is divided into two chambers or pump sumps (4.o a and 4.o b), wherein a separate downward run (17a, 17b) for the contaminated washing liquid belongs to each of these pump sumps and that the overflows (8a and 8b) of the hydrocyclone stages (9a, 9b) are conducted each time into the associated pump sump (4.o a or 4.o b).

**22.** Method for the operation of an arrangement according to one of claims 1 to 21, characterised thereby that the feed of washing liquid (15) and in a given case the feed of a filtrate (16) and the output of the pumps (11a to 11e) are matched to one another in such a manner that there are always more liquid feeds (7b to 7f; 13a to 13f; 8c to 8f; 15 and in a given case 16) to the individual pump sumps (4a to 4f) than pump outlets.

**23.** Method according to claim 22, characterised thereby, that the quantity fed per unit time and the clean washing liquid forming the upstream flow are so adjusted that possible crystallisations of the terephthalic acid are dissolved at least to such an extent that their size is equal to or smaller than a determined maximum size.

**24.** Method according to claim 23, characterised thereby, that the feed quantity to the upstream liquid is regulated or controlled in dependence on the respective suspension as well as the degree of contamination thereof or crystallisation arising there.

**25.** Method according to claim 23 or 24, characterised thereby, that the degree of dissolution and/or the particle size being separated out are influenced by measuring the density of the suspension which is disposed in the bed (39) of the upstream classifier and by corresponding change of the washing water quantity.

**26.** Method according to claim 23 or 24, characterised by a regulating or controlling of the suspension feed.

**27.** Method according to claim 26, characterised thereby, that in the case of constant grain size of the components of the suspension feed a regulation of the suspension feed takes place in dependence on its density.

**28.** Use of an arrangement according to one of the claims 1 to 21 and/or of a method according to one of claims 22 to 27 for the treatment of saturated solutions with suspended solids which crystallise out, for the purpose of avoiding crystallisation.

**29.** Use according to claim 28 in the case of a method for the production of terephthalic acid.

**30.** Use according to claim 28 in the case of a method for the production of pure terephthalic acid from dimethylterephthalate as intermediate product.

**31.** Use according to claim 29 in the case of a method for the production of pure terephthalic acid from raw terephthalic acid as intermediate product.

32. Use of an arrangement according to one of claims 1 to 21 and/or of a method according to one of claims 22 to 27 for the treatment of a hydrocarbon suspension containing ash-containing fine coal with a grain size preferably smaller than 1 millimetre, wherein water is provided as washing liquid and the overflow represents the desired product in the form of the further hydrogenatable fine coal fraction suspended in hydrocarbonaceous liquid.

**Revendications**

1. Dispositif à plusieurs étages pour le lavage à contre-courant de solides, et de préférence de cristaux, qui sont en suspension, à l'aide d'hydrocyclones (9a - 9f) montés les uns derrière les autres, ainsi que de pompes associées (11 - 11e), de réservoirs de pompage (4 - 4f) et de conduites reliant les éléments constitutifs précités, cependant qu'il est prévu des amenées destinées au liquide de lavage (15) et aux solides en suspension (1), ainsi que des sorties destinées au liquide de lavage chargé d'impuretés (17 ; 17a, 17b) et à la suspension lavée et épaissie (14), qu'en outre le liquide de lavage est déplacé à contre-courant (F2) par rapport à la direction (F1) du déplacement des solides en suspension, et que les solides en suspension sont traités sous une pression et une température élevées, caractérisé par le fait que les hydrocyclones (9a - 9f) montés les uns derrière les autres, ou des groupes montés les uns derrière les autres et constitués par plusieurs hydrocyclones (9a - 9f) montés en parallèle (tous deux appelés seulement "groupes d'hydrocyclones" dans ce qui suit), sont logés avec des sous-verses (13a - 13f) et des surverses (8a - 8f) dans une cuve commune (18) résistant à la pression et calorifugée, et que les réservoirs de pompage (4 - 4f) y sont logés avec les pompes (11 - 11e) ou les parties de pompe qui y pénètrent, et par le fait que cette cuve est munie de l'amenée (1) destinée aux solides en suspension, d'une amenée (15) et d'une sortie (17) destinées au liquide de lavage et d'une sortie (14) destinée à la suspension lavée et épaissie.

2. Dispositif selon la revendication 1, caractérisé par le fait que les parties ou les régions importantes des conduites sont également logées dans la cuve commune (18) résistant à la pression et calorifugée.

3. Dispositif selon la revendication 1, caractérisé par le fait que des parties ou des régions importantes des conduites, ou des parties des pompes ou des conduites de pompage sont prévues à l'extérieur de la cuve (18) résistant à la pression et calorifugée, et qu'elles sont calorifugées et/ou chauffées de l'extérieur, pour autant qu'elles conduisent les agents du lavage à contre-courant qui doivent être maintenus à l'intérieur d'une gamme de températures déterminée.

4. Dispositif selon l'une des revendications 1 à 3, caractérisé par le fait que la cuve (18) est munie de coupoles (19a - 19f) qui sont résistantes à la pression et calorifugées et qui comprennent des couvercles associés amovibles (20a - 20f), les hydrocyclones appartenant à un groupe (9a - 9f) étant fixés au couvercle correspondant et logés dans la coupole correspondante.

5. Dispositif selon la revendication 4, caractérisé par le fait que les hydrocyclones ou, respectivement, les groupes d'hydrocyclones (9a - 9f) sont fixés dans la coupole au moyen de pièces extensibles, et par exemple de soufflets en accordéon (44).

6. Dispositif selon l'une des revendications 1 à 5, caractérisé par le fait que les parois de la cuve et, le cas échéant, des coupoles peuvent être chauffées de l'extérieur (25).

7. Dispositif selon l'une des revendications 1 à 6, caractérisé par le fait que les surverses (8a - 8f) et les sous-versez (13a - 13f) des hydrocyclones ou des groupes d'hydrocyclones (9a - 9f), respectivement, sont en communication avec les réservoirs de pompage (4 - 4f) à l'intérieur de la cuve (18) ou des coupoles (19a - 19f) de celle-ci.

8. Dispositif selon l'une des revendications 1 à 7, caractérisé par le fait qu'au moins les moteurs des pompes (11 à 11e) et, partiellement, les conduites de refoulement (30 à 30e) des pompes, se trouvent à l'extérieur de la cuve (18), les conduites de refoulement des pompes étant reliées aux amenées (12a à 12f) des groupes d'hydrocyclones (9a à 9f) qui sont les premiers à suivre dans la direction (F1) du déplacement des solides en suspension.

9. Dispositif selon l'une des revendications 1 à 8, caractérisé par le fait qu'est associé à chaque réservoir de pompage (4c à 4f) un agitateur (6c à 6f) dont le moteur (29c à 29f) se trouve à l'extérieur de la cuve (18).

10. Dispositif selon l'une des revendications 1 à 9, caractérisé par le fait que, dans une cuve allongée (18), il est prévu, dans la région d'une extrémité, l'amenée (1) des solides en suspension et la surverse d'évacuation (17) du liquide de lavage, ainsi que, dans la région de l'autre extrémité, l'amenée (15) du liquide de lavage et la sortie ou, respectivement, l'évacuation (14) de la suspension de solides lavée et épaissie, les étages de l'installation de lavage à contre-courant se trouvant entre les régions des extrémités en étant montés les uns derrière les autres.

11. Dispositif selon la revendication 10, caractérisé par le fait que, dans les étages (a à e) de la cuve (18), sont disposés l'un à côté de l'autre, c'est-à-dire dans un plan à peu près perpendiculaire au plan médian longitudinal (31) de la cuve, à chaque fois un groupe d'hydrocyclones (9a à 9f), ou la coupole qui contient ce groupe d'hydrocyclones, et à chaque fois une pompe (11 à 11e) munie d'un agitateur (6a à 6e), cependant que le réservoir de pompage (4a à 4e), lequel est séparé par des cloisons (28a à 28f) des réservoirs de pompage qui sont à chaque fois voisins, se trouve dans la zone inférieure de chaque étage.

12. Dispositif selon la revendication 11, caractérisé par le fait que la sous-verse (13a à 13f) du groupe d'hydrocyclones (9a à 9f) de chaque étage s'écoule directement vers le bas dans le réservoir de pompage (4a à 4f) qui se trouve au-dessous.

13. Dispositif selon la revendication 11 ou 12, caractérisé par le fait que la surverse (8a à 8f) du groupe d'hydrocyclones (9a à 9f) de chaque étage s'écoule directement vers le bas dans une rigole, une goulotte ou similaire (27a à 27f) qui est inclinée et disposée d'une manière telle qu'elle fasse s'écouler la surverse, dans la direction (F2) du déplacement du liquide de lavage et selon une pente naturelle, vers le réservoir de pompage qui est le deuxième à suivre ou, respectivement, vers le réservoir de pompage (4o) de la sortie du liquide de lavage.

14. Dispositif selon l'une des revendications 11 à 13, caractérise par le fait que, dans les sections ou, respectivement, les étages de la cuve (18) qui se trouvent les uns derrière les autres dans la direction longitudinale de celle-ci, les pompes (11 à 11e) et les groupes d'hydrocyclones (9a à 9f) sont disposés des deux côtés d'un plan médian longitudinal (31) de la cuve, cependant qu'il est prévu, en alternance dans la direction longitudinale de la cuve, une pompe et un hydrocyclone ou, respectivement, la coupole qui les reçoit, et par le fait que la conduite de refoulement (30 à 30e) d'une pompe est à chaque fois reliée à l'amenée (12a à 12f) du groupe d'hydrocyclones ou de la coupole, respectivement, qui est voisine dans la direction (F1) du déplacement des solides en suspension.

15. Dispositif selon l'une des revendications 11 à 14, caractérisé par le fait que la hauteur des cloisons (28a à 28f) qui sont placées entre les réservoirs de pompage décroît dans la direction (F2) du déplacement du liquide de lavage, et qu'elle permet donc un trop-plein d'un réservoir de pompage au réservoir de pompage qui est voisin dans cette direction de déplacement (F2).

16. Dispositif selon l'une des revendications 1 à 15, caractérisé par le fait qu'il est prévu, dans le cours du lavage à contre-courant, un classificateur à courant ascendant (32) destiné à éliminer d'éventuelles cristallisations et/ou des particules solides plus grossières, et qu'il est également logé dans la cuve commune (18) résistant à la pression et calorifugée.

17. Dispositif selon la revendication 16, caractérisé par le fait que la zone inférieure du classificateur à courant ascendant (32), laquelle comporte l'amenée du liquide de lavage et la sortie d'extraction (43), est disposée dans une coupole (33, 34) de la cuve, le couvercle (34) de la coupole étant amovible, et par le fait que cette coupole est également résistante à la pression et calorifugée.

18. Dispositif selon la revendication 16 ou 17, caractérisé par le fait que, dans un procédé pour la fabrication d'acide téréphtalique, le classificateur à courant ascendant (32) est prévu à l'entrée (15) du liquide de lavage, par le fait que l'amenée (15) du liquide de lavage propre est reliée à l'ouverture de courant ascendant de celui-ci en constituant le courant ascendant du classificateur, par le fait que la sous-verse (13e) de l'hydrocyclone associé ou, respectivement, du groupe d'hydrocyclones associé (9e) qui se trouve au-dessus, est amenée à l'entrée du classificateur à courant ascendant, par le fait que le trop-plein (42) du classificateur à courant ascendant s'écoule dans le réservoir de pompage (4e) qui lui appartient, et qu'il continue à progresser depuis cet endroit, et par le fait que la sortie d'extraction (43) du classificateur à courant ascendant est fermée.

**19.** Dispositif selon la revendication 16 ou 17, caractérisé par le fait que, pour le traitement de solutions saturées contenant des solides en suspension qui peuvent cristalliser, ou pour le traitement de fines de charbon contenant des cendres, le classificateur à courant ascendant (32) est de préférence prévu dans la cuve à la sortie du liquide de lavage chargé d'impuretés, par le fait que son courant ascendant est constitué par un liquide de lavage (45) qui est amené séparément, et par le fait que la sortie d'extraction (43) du classificateur à courant ascendant est ouverte en vue de la dérivation des cristaux ou des particules solides qui ont été classifiées.

**20.** Dispositif selon la revendication 18 ou 19, caractérisé par le fait que le classificateur à courant ascendant (32) comporte une enveloppe (40) munie d'un bord de tropplein (41) et d'un fond (34), et qu'il est disposé au-dessous de la sous-verse (13a à 13e) d'un hydrocyclone ou, respectivement, d'un groupe d'hydrocyclones, par le fait que c'est dans le fond (34) que se trouvent l'amenée (15) destinée au liquide de lavage et la tubulure d'extraction (43), et par le fait que le trop-plein (42) du classificateur à courant ascendant (32) s'écoule sur le bord de trop-plein (41) au-dessus du réservoir de pompage qui lui est associé.

**21.** Dispositif selon l'une des revendications 1 à 20, caractérisé par le fait que l'étage (o) qui est situé à l'extrémité de la cuve dans la direction d'écoulement du liquide de lavage est divisé en deux chambres ou, respectivement, en deux réservoirs de pompage (4.o a et 4.o b), une sous-verse séparée (17a, 17b) de liquide de lavage chargé d'impuretés appartenant à chacun de ces réservoirs de pompage, et par le fait que les surverses (8a et 8b) des étages d'hydrocyclones (9a, 9b) sont amenées à chaque fois dans le réservoir de pompage associé (4.o a et, respectivement, 4.o b).

**22.** Procédé pour le fonctionnement d'un dispositif selon l'une des revendications 1 à 21, caractérisé par le fait que l'amenée (15) du liquide de lavage et, le cas échéant, l'amenée (16) d'un produit de filtrage, et la capacité des pompes (11a - 11e) sont harmonisées entre elles d'une manière telle qu'il y ait toujours plus de liquide amené (7b - 7f ; 13a - 13f ; 8c - 8f ; 15 et, le cas échéant, 16) aux divers réservoirs de pompage (4a - 4f) qu'il n'en est extrait par les pompes.

**23.** Procédé selon la revendication 22, caractérisé par le fait que la quantité du liquide de lavage pur qui est amenée par unité de temps et qui constitue le courant ascendant est réglée d'une manière telle que des cristaux éventuels d'acide téréphtalique soient du moins suffisamment dissous pour que leur taille soit égale ou inférieure à une taille maximale déterminée.

**24.** Procédé selon la revendication 23, caractérisé par le fait que la quantité de liquide amenée dans le courant ascendant est régulée ou commandée en fonction de chaque suspension, ainsi que de son taux d'impuretés, ou, respectivement, de la cristallisation qui prend naissance à cet endroit.

**25.** Procédé selon la revendication 23 ou 24, caractérisé par le fait que l'on agit sur le degré de dissolution et/ou sur la taille des particules à séparer en mesurant la densité de la suspension qui se trouve dans le lit (39) du classificateur à courant ascendant, et en modifiant en conséquence le débit de l'eau de lavage.

**26.** Procédé selon la revendication 23 ou 24, caractérisé par une régulation ou une commande de l'amenée de la suspension.

**27.** Procédé selon la revendication 26, caractérisé par le fait que, dans le cas d'une granulométrie constante des constituants de la suspension qui est amenée, une régulation de l'amenée de la suspension a lieu en fonction de sa densité.

**28.** Utilisation d'un dispositif selon l'une des revendications 1 à 21, et/ou d'un procédé selon l'une des revendications 22 à 27, pour le traitement de solutions saturées contenant des solides en suspension qui cristallisent, en vue d'éviter la cristallisation.

**29.** Utilisation selon la revendication 28 dans un procédé pour la fabrication d'acide téréphtalique pur.

**30.** Utilisation selon la revendication 29 dans un procédé pour la fabrication d'acide téréphtalique pur à partir de diméthyltéréphtalate servant de produit intermédiaire.

**31.** Utilisation selon la revendication 29 dans un procédé pour la fabrication d'acide téréphtalique pur à partir d'acide téréphtalique brut servant de produit intermédiaire.

**32.** Utilisation d'un dispositif selon l'une des revendications 1 à 21, et/ou d'un procédé selon l'une des revendications 22 à 27, pour le traitement d'une suspension dans un hydrocarbure de fines de charbon qui contiennent des cendres et dont la granulométrie est de préférence inférieure à 1 mm, cependant que de l'eau est prévue comme liquide de lavage et que la surverse, sous la forme de la fraction de fines de charbon qui est en suspension dans le liquide contenant un hydrocarbure et qui peut continuer à être hydrolysée, représente le produit visé.

Fig.1

Fig.2

Fig.3

# Fig.4

# Fig.5

Fig.6

Fig.7

Fig.8

Fig.9